(19) Europäisches Patentamt
European Patent Office
Office européen des brevets

(11) **EP 3 941 205 B1**

(12) **EUROPEAN PATENT SPECIFICATION**

(45) Date of publication and mention
of the grant of the patent:
**03.09.2025 Bulletin 2025/36**

(21) Application number: **20710176.7**

(22) Date of filing: **16.03.2020**

(51) International Patent Classification (IPC):
*A01N 63/27* (2020.01)    *C12R 1/38* (2006.01)
*C12N 1/20* (2006.01)    *A01P 1/00* (2006.01)
*A01P 3/00* (2006.01)

(52) Cooperative Patent Classification (CPC):
**A01N 63/27; C12N 1/20; C12N 1/205; C12P 1/04;**
C12R 2001/38

(86) International application number:
**PCT/EP2020/057051**

(87) International publication number:
**WO 2020/187822 (24.09.2020 Gazette 2020/39)**

(54) **PSEUDOMONAS SP. STRAIN, COMPOSITION COMPRISING THE SAME, AND USES THEREOF**

PSEUDOMONAS SP.STAMM, ZUSAMMENSETZUNG DAMIT UND VERWENDUNGEN DAVON

SOUCHE DE PSEUDOMONAS SP.,COMPOSITION LA COMPRENANT ET SES UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **18.03.2019 EP 19382190**

(43) Date of publication of application:
**26.01.2022 Bulletin 2022/04**

(73) Proprietor: **Futureco Bioscience, S.A.
08799 Olèrdola - Barcelona (ES)**

(72) Inventors:
• **FERNÁNDEZ CASTILLO, Carolina
08799 Olèrdola (ES)**
• **LARA SÁNCHEZ, José Manuel
08799 Olèrdola (ES)**

(74) Representative: **ZBM Patents - Zea, Barlocci &
Markvardsen
Rambla de Catalunya, 123
08008 Barcelona (ES)**

(56) References cited:
**EP-A1- 2 654 433    WO-A1-2017/178529
WO-A1-94/03065    CN-A- 107 119 000
US-A- 5 232 850**

Remarks:
The complete document including Reference
Table(s) and the Sequence Listing(s) can be
downloaded from the EPO website

**Description**

This application claims the benefit of European Patent Application 19382190.7 filed on March 18th, 2019.

**Technical Field**

The present invention relates to the field of phytosanitary products. It provides a new isolated strain of the microorganism *Pseudomonas sp.* and compositions comprising the same and their use in the biological control of diseases caused by bacterial and/or fungal infections in plants.

**Background art**

Chemical pesticides are known to contaminate the environment. They have been used on crops across the world for several years, creating an accumulation of adverse pollution in our environment, which continues to grow with every application. Studies have demonstrated that chemical pesticides persist in the ground, the waterways and the atmosphere long after they have been used.

Unfortunately, when chemical pesticides are applied onto a surface, they travel outside their intended area of use by air, soil or water. This can lead to pollution of surface water and groundwater, reduced pest control, and damaging of non-target species, including humans.

In addition, chemical pesticides not only contaminate food, but they also drain the nutritional value of it. Research has consistently reported chemical pesticide residues in a third of food, including lettuces, potatoes, peaches, strawberries, apples, cereal bars, baby food, bread, fresh salmon, and lemons. Despite pesticides are designed to kill living organisms, they are certainly not meant to enter human bodies. However, they have been linked to a myriad of diseases.

Luckily, from time ago the use of biocontrol agents has become more and more established to avoid the undesirable effects of the chemical pesticides. This kind of pesticides, the biopesticides, has been reported to decrease the diseases intensity, play a key role in integrated management of diseases, be more selective and effective than conventional pesticides, be safer for the users and the farming community, and provide a natural long term immunity to crops and soil.

In this context, in the last 15 years the use of biocontrol agents has increased four times in crop plants. Such use can be seen with *Pseudomonas* strains in WO2017/178529A1, US5232850A and WO94/03065A1 for example.

However, it is worth mentioning that the host and environmental conditions affect the biological activity of the bacteria strains, making their effectiveness sometimes lower and variable than the reference chemical products.

Therefore, in spite of the efforts made, there is a need for finding new biocontrol agents.

**Summary of the invention**

The inventors have surprisingly found one strain of the *Pseudomonas sp.* (hereinafter also referred as "strain B2021") which has a remarkable pesticide activity against both bacteria and fungi as shown in Example 4. In addition, the inventors have also found that the strain of the invention has surfactant activity which, on one hand, contributes to the high anti-bacterial and fungal activity of the strain of the invention, and, on the other hand, may provide beneficial effects not only to the plants to be protected but also to the soil where they are grown.

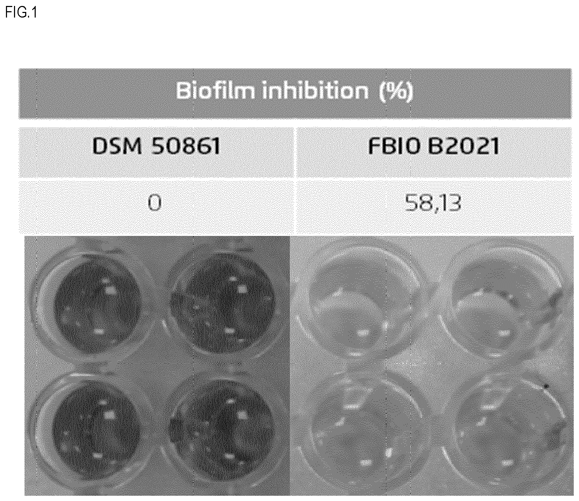

As it is shown below, the strain of the invention has been tested in several assays to prove its antibacterial effect. Thus, the biofilm formation inhibition assay to validate its capacity to inhibit the biofilm formation of a strain of *Xanthomonas vesictoria* (Example 4A and FIG.1); as well as bioassays on potato and tomato plants against an *Erwinia carotovora* strain (Example 4C; Table 15) and a *Pseudomonas syringae pv* tomatoExample 4D; Table 17), respectively, were performed. In all cases, the strain of the invention demonstrated to be efficient in inhibiting the bacterial growth and when compared with commercially available biocontrol agents it exhibited even better results.

As mentioned, the strain of the invention has also anti-fungal effect as shown in Examples 4B and 4F. The strain B2021 inhibited a 50% the severity of the *Rhizoctonia solani* infection on a potato plant, having an efficacy equivalent to the commercially available chemical pesticide Cuproflow.

The inventors have also found that the strain of the invention has the ability to produce and secrete to the medium siderophores and surfactant metabolites (Example 2 and 3). The inventors have also found that these secreted metabolites contribute to the dual antibacterial and antifungal effect (Example 4E and 4F).

In addition, to such outstanding activity profile, it was also found that the strain of the present invention did not present acute oral toxicity *in vivo* in rats. The results of Example 6 show that animals had no visible clinical adverse effects and no infectivity of pathogenicity was found. Without being bound to the theory it is believed that this can be at least due to the fact that the strain of the invention has an optimal temperature growth range of 28°C-30°C

The biological nature of the strain makes it, when used as pesticide, environmentally friendly.

In addition, the strain of the invention is mesophile and neutrophile, is capable of growing from 0% to 4% of NaCl and it is considered a non-halotolerant strain. These features contribute to the ability of the strain to efficiently colonize the soil.

Furthermore, it shows sensitivity to oxytetracycline and is highly resistance to Streptomycine sulfate (Table 8).

Altogether the strain of the present invention means a great advance in the field of biopesticides.

When the present inventors performed the genomic characterization of the strain of the invention (by (ANI analysis), they found that it belonged to *Pseudomonas* genus and that it was not coincident to any of the already known species but only close to *Pseudomonas fluorescens* and *Pseudomonas azotoformans.*

In particular, when strain's genome sequence SEQ ID NO: 1 was aligned to different genomes of strains of *P. fluorescens, P. azotoformans,* and *P. yamanorum* for ANI calculation, it was found that ANIb and ANIm values were lower than 89%. The established cut-off used to confirm that two strains belong to the same species corresponds to an ANI of 95 ± 0.5%. That is, the strain of the invention pertains to a new specie, which has not been reported before.

Thus, the first aspect of the present invention refers to a *Pseudomonas sp.* strain deposited at the "Colección Española de Cultivos Tipo" (CECT) under the accession number CECT8708 (the strain of the invention).

In the present invention the terms "B2021" and "CECT8708", referring to the strain of the invention, are used interchangeably.

The strain of the invention was isolated from an agricultural soil in Almeria (Spain) and was deposited, according to the Budapest Treaty, at the CECT in the Universidad de Valencia C.P 46980 Catedrático Agustin Escardino N° 9 Paterna, Valencia (Spain), under the accession number CECT8708. It was deposited by the depositor Fututeco Bioscience S.A., Av. Del Cadi 19-23 P.I. Sant Pere Molanta 08799 Olèrdola Barcelona, on the 23rd September 2014. The strain was identified by the depositor with the reference B2021, and received the accession number CECT8708. It was, in addition, declared viable.

A second aspect of the present invention refers to a process for obtaining a viable cell suspension derived from the strain as defined in the first aspect, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.

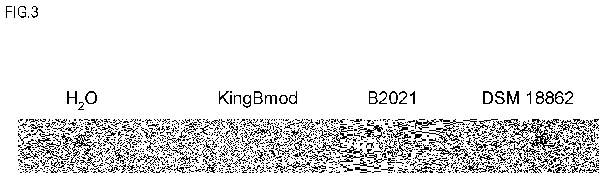

As shown below the cell-free extract (CS) fraction of the invention shows the antibacterial and antifungal capacities of the cell extract including the strain of the invention. This matches with the finding that the strain produces and secretes siderophores and surfactant (Examples 2 and 3, table 11, FIG. 3).

In Examples 3 and 4F, the cell-free extract from a *Pseudomonas azotoformans* strain and the one of the invention were compared in terms of components and antifungal activity. As shown below, the antifungal activity of the CS of the invention was higher than the one achieved with the CS derived from the *Pseudomonas azotoformans* strain. In addition, it was found that only the CS of the invention was able to produce surfactant (FIG.4).

This is indicative that the strain of the invention produces a well-differentiated CS.

The inventors have found that the cell-free extract of the invention comprises pyoverdin-type siderophores as well as surfactant metabolites.

Thus, a third aspect of the present invention refers to a process for obtaining a cell-free extract derived from the strain as defined in the first aspect, said process comprising: (i) inoculating the strain in a suitable culture medium, (ii) subjecting the inoculated culture medium to suitable growth conditions, (iii) separating the cells from the culture medium of step (ii), (iv) collecting cell-free extract; and (v) optionally subjecting the cell-free extract to a concentration process.

A fourth aspect of the present invention refers to an inoculation product comprising the strain as defined in the first aspect.

Due to the pesticidal effect of the strain of the present invention, it can be used as a phytosanitary product. As it is illustrated in the examples below, the strain of the first aspect of the can be used as a phytosanitary product according to FAO guidelines, which are the guidelines emitted by Food and Agriculture Organization of the United Nations. The isolated bacteria as defined in the first aspect of the invention may be used as ingredient of a phytosanitary composition.

Therefore, a fifth aspect of the present invention refers to a composition comprising the strain as defined in the first aspect or an inoculation product as defined in the fourth aspect of the invention, and one or more agriculturally acceptable compound(s).

A seventh aspect of the present invention refers to the use of the strain as defined in the first aspect of the invention, or the inoculation product as defined in the fourth aspect of the invention, or the composition as defined in the fifth aspect of the invention as a pesticide in plants.

An eighth aspect of the present invention relates to a method for controlling an infection caused by bacterial or fungal pathogen in a plant, comprising applying the strain as defined in the first aspect of the invention, or the inoculation product as defined in the fourth aspect of the invention, or the composition as defined in the fifth aspect of the invention, to the affected part of the plant and/or to the seed or to the substrate or soil used for growing said plant.

A ninth aspect of the present invention relates to a kit comprising an effective amount of the strain as defined in the first aspect of the invention, or the inoculation product as defined in the fourth aspect of the invention, or the composition as defined in the fifth aspect of the invention.

A tenth aspect of the invention refers to the use of the kit of the ninth aspect of the invention for controlling an infection caused by bacterial pathogens and/or fungal pathogens in a plant.

**Brief description of the drawings**

Fig. 1 shows the results of the biofilm inhibitory assay described in Example 4A.

Fig. 2 HPLC chromatograms at 254 nm of CS produced by B2021 (A) and DSM 18862 (B). Each chromatogram corresponds to KingBmod culture medium (black line), bacterial incubation in KingBmod medium (dashed line) and bacterial incubation in medium supplemented with iron (dotted line)

Fig. 3 Surfactant activity of CS produced by the strain of the invention B2021 and the *Pseudomonas azotoformans* strain DSM 18862 compared with $H_2O$ and the KingBmod culture medium as controls.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

The first aspect of the invention refers to an isolated strain of a *Pseudomonas sp.* strain deposited at the "Colección Española de Cultivos Tipo" (CECT) under the accession number CECT8708.

"Average Nucleotide Identity" also referred herein as ANI is a descriptor of genetic relatedness. This descriptor is derived from lineage-specific genes and widely distributed genes (typically> 1000 genes in total), thereby increasing the robustness and resolution of extracted phylogenetic signals. Furthermore, since ANI is based on a large number of genes, it is a better measure of relatedness than data derived from a single gene, such as the 16S rRNA gene. In addition, varied evolutionary rates or horizontal gene transfer of single or a few genes do not significantly affect ANI values, since the effect of fast-evolving genes is mitigated by the slow evolution of other genes. According to several studies, the recommended cut-off point of 70% DNA-DNA hybridization (DDH) data for species delineation thereby corresponded to an ANI of $95\pm 0.5\%$ (Arahal et al., Methods in Microbiology, Chapter 6, Whole genome analysis: Average Nucleotide Identity", Vol. 41 ISSN 0580-9517).

To run this kind of analysis, one has to perform whole genome sequencing of the strain of interest. To do so, the genomic DNA has to be extracted and quantified. To extract sufficient high quality DNA for whole genome sequencing, many different methods including conventional procedures and the use of commercial kits, can be carried out. After that, whole genome sequencing can be performed with high-throughput DNA sequencing technology using benchtop instruments such as, but not limited to, 454 GS FLX Titanium/GS Junior (Roche), Genome Analyzer/HiSeq 2000/MiSeq (Illumina), SOLiD/Ion Torrent PGM (Life Technologies), or RS (Pacific Biosciences).

ANI calculations can be done using the JSpecies server (http://jspecies.ribohost.com/jspeciesws). To calculate species relationships two additional software packages need to be installed which are BLAST (ftp://ftp.ncbi.nih.gov/-blast/executables/) and MUMmer (http://mummer.sourceforge.net). BLAST search algorithm follows a hashing approach that involves the generation of a large number of short sequence stretches and character comparisons, whereas MUMmer makes use of suffix trees to find potential anchors for an alignment. The BLAST calculation of ANI values, using the Blast algorithm, is called ANIb and ANI values calculated by using the NUCmer program in the MUMmer software package are called ANIm.

As shown below, the inventors have carried out the genomic characterization of the strain of the invention. To that aim, first, 16S rRNA gene sequence was amplified by PCR, sequenced and aligned with the BLAST *suite* to find out the species of the strain of the invention. The parameters used to analyse the 16S rRNA sequence were those established by default except for the following: the Database: Nucleotide collection (nr / nt); the Organism: Pseudomonas (taxid: 286); Limit to: Sequences from type material; and Optimized for: Somewhat similar sequences (blastn). According to their results, the strain of the invention had a 99-100% identity to the 16S rRNA gene of three different *Pseudomonas azotoformans* strains, two *Pseudomonas mucidolens* strains, and two *Pseudomnas synxantha* (Table 1). Although the 16S rRNA has been widely used for species identification, in the *Pseudomonas* genus this criterium is not enough to identify a species since, as can be extracted also from the results obtained, there are several species that share common 16S rRNA gene sequence.

**EP 3 941 205 B1**

Next, rpoD, gyrB and gyrA genes sequences were obtained from the whole genome sequence and analysed with the BLAST *suite* using the same parameters as 16S rRNA analysis. Regarding rpoD gene, the closest sequences belonged to three different species of *Pseudomonas,* the *P. fluorescens* ATCC13525, the *P. azotoformans* LMG21611, and the *P. yamanorum* LMG2747 (Table 2). Regarding gyrB gene, the closest sequences belonged to three different species of *Pseudomonas* too, the *P. synxantha* LMG2190, the *P. azotoformans* LMG21611, and the *P. fluorescens* ATCC13525 (Table 3). Finally, in the case of the gyrA gene, the closest sequences belonged to three different species of *Pseudomonas* too, the *P. azotoformans* LMG21611, the *P. synxantha* LMG2190, and the *P. extremaustralis DSM* 17835 (Table 4). Taking into account the results of this homology analysis, there were three species which were considered to be the more closely related with the strain of the invention that are *P. fluorescens* ATCC13525, the *P. azotoformans* LMG21611, and the *P. yamanorum* LMG2747, as they appeared in the results of all the genes examined in the first five positions.

To further dissect the species of the strain of the invention, the genomes of the strains identified to be more closely related *P. fluorescens* ATCC13525, *P. azotoformans* LMG21611, *P. fluorescens* NCTC_10038, *P. yamanorum* LMG2747, *P. azotoformans* S4, *P. azotoformans* F77, and the one from the strain of the invention (SEQ ID NO: 1) were compared using ANIb and ANIm calculation using the parameters by default appearing in the online tool (Table 5 to 7).

As shown in Example 1C, the inventors did not find any species with an ANIb higher than 86.24, and an ANIm higher than 88.38, which indicated that the strain of the invention did not belong to any of those species and, thus, that it has not been reported before.

The term "about" or "around" as used herein refers to a range of values ± 10% of a specified value. For example, the expression "about 10" or "around 10" includes ± 10% of 10, i.e. from 9 to 11.

In the present invention the terms "B2021" and "CECT8708", referring to the strain of the invention, are used interchangeably.

The strain of the first aspect of the invention was isolated from an agricultural soil in Almeria (Spain) and was deposited, according to the Budapest Treaty, at the CECT in the Universidad de Valencia C.P 46980 Catedrático Agustin Escardino N° 9 Paterna, Valencia (Spain), under the accession number CECT8708. It was deposited by the depositor Fututeco Bioscience S.A., Av. Del Cadi 19-23 P.I. Sant Pere Molanta 08799 Olèrdola Barcelona, on the 23rd September 2014. The strain was identified by the depositor with the reference B2021, and received the accession number CECT8708. It was, in addition, declared viable.

The inventors have genomically characterized the strain of the invention and have identified this strain to belong to a species not reported before. Being the *Pseudomonas fluorescens* and *Pseudomonas azotoformans* the more closely related taking into account the comparative analysis performed (Example 1).

The dual profile as anti-bacterial and anti-fungal of the strain of the invention may be determined following several well-known protocols. These methods are commonly based on the analysis of the growth capacity of the pathogen in contact with the strain, or the assessment of the severity of the disease caused by the pathogen infection after the exposition to the strain. The protocols that are included in the examples herein for the determination of the activity are illustrative and non-limitative examples.

In an embodiment of the first aspect of the invention, optionally in combination with any of the embodiments provided above or below, the bactericidal effect is at least against bacteria of the genus *Xanthomonas, Pseudomonas* and *Erwinia.* In another embodiment, the bactericidal effect is against one or more of *Xanthomonas vesicatoria, Pseudomonas syringae,* and *Erwinia carotovora.* In another embodiment, the strain as defined in the first aspect of the invention has a bactericidal effect against *Xanthomonas vesicatoria, Pseudomonas syringae,* and *Erwinia carotovora.*

In another embodiment of the first aspect of the invention, optionally in combination with any of the embodiments provided above or below, the strain as defined in the first aspect of the invention has a fungicidal effect at least against fungi of the genus *Rhizoctonia* and *Botrytis.* In a further embodiment, optionally in combination with any of the embodiments provided above or below, the strain as defined in the first aspect of the invention has a fungicidal effect against one or more of *Rhizoctonia solani* and *Botrytis aclada.* In another embodiment, the strain as defined in the first aspect of the invention has a fungicidal effect against *Rhizoctonia solani* and *Botrytis aclada.*

In another embodiment of the first aspect of the invention, optionally in combination with any of the embodiments provided above or below, the strain as defined in the first aspect of the invention has a bactericidal effect at least against bacteria of the genus *Xanthomonas, Pseudomonas,* and *Erwinia;* and a fungicidal effect at least against fungi of the genus *Rhizoctonia* and *Botrytis.* In a further embodiment, optionally in combination with any of the embodiments provided above or below, the strain as defined in the first aspect of the invention has a bactericidal effect against *Xanthomonas vesicatoria, Pseudomonas syringae,* and *Erwinia carotovora*; and a fungicidal effect against *Rhizoctonia solani* and *Botrytis aclada.*

In a second aspect, the present invention provides a process for obtaining a viable cell suspension derived from the strain as defined in the first aspect of the invention, the process comprising: (i) inoculating the strain in a culture medium, (ii) subjecting the inoculated culture medium of step (i) to conditions suitable for growth of the strain, and (iii) optionally subjecting the medium resulting from step (ii) to a concentration step.

The term "derived from the strain of the invention" means that the suspension is obtained from the strain object of the present invention.

The strain of the invention may be inoculated in the culture medium at a final concentration comprised from 5 to 7% v/v. Preferably the inoculated culture is in an exponential growth phase. Cell growth will slow on achieving, preferably, a cell concentration comprised from $1{,}0x10^9$ to $1{,}0x10^{10}$ CFU/mL. Suitable culture media for the growth of the strain of the invention are synthetic media, such as LB (lysogenic broth) and PM (saline production medium), or media of plant origin such as molasses (e.g. from sugar cane, beets and others). Suitable conditions for strain's growth are temperatures comprised from 24 to 30 °C, pH comprised 6 to 8, and oxygen concentration comprised from 50 to 100%. The growth of the strain of the invention is produced by stirring. An example of the detailed method for obtaining cells from the strain of the invention is reflected in Example 5.

In another embodiment of the process for obtaining the suspension, cells are separated from the medium to obtain a concentrated suspension. Suitable separation techniques include centrifugation or filtration of the culture. Carrying out the centrifugation of the culture, for example, at a minimum of 7000 rpm, cells are obtained in the pellet, which are resuspended in part of the culture medium or in a suitable buffered medium such that the strain concentration is approximately about $5x10^{10}$ CFU/mL.

Once the suspension is obtained, it may be subjected to a dehydration step. Dehydration can be carried out through a lyophilisation process. Alternatively, the suspension can be dehydrated by fluidised bed drying. Another option is to dehydrate the suspension by spray drying or drying in an oven under vacuum. In order to improve cell viability, an inert osmotic protector ingredient can be added to the suspension before carrying out the dehydration process.

In another embodiment of the second aspect of the invention, the process comprises resuspending the cells resulting from the separation step in a suitable buffer to yield a cell concentrated suspension.

As mentioned above, the CS extract from the strain of the invention has been proved to maintain the anti-bacterial and anti-fungi activities (Example 4E and 4F). In this regard, the inventors have found that these effects are related to the siderophore production by the strain of the invention. As shown in Example 4E and 4F, when the CS extract is obtained in the presence of iron, that is, no siderophores can be produced, the anti-microbial effect of the strain of the invention is importantly reduced (Table 18 and 19).

In a third aspect, the present invention provides a process for obtaining a cell-free extract derived from the strain as defined in the first aspect of the invention, said process comprising: (i) inoculating the strain in a suitable culture medium; (ii) subjecting the inoculated culture medium to suitable growth conditions; (iii) separating the cells from the culture medium of step (ii); (iv) collecting the cell-free extract; and (v) optionally subjecting the cell-free extract to a concentration step.

As commented above and shown below, the activity profile of the cell-free extract activity may be modulated on the basis of the composition of the culture medium. In one embodiment of the third aspect, optionally in combination with any of the embodiments provided above or below, the suitable culture medium is free of iron. Advantageously, in an iron-free medium, the strain of the invention is capable of producing siderophores.

The cell-free medium obtained by the separation processes described above, could be used and/or included in an appropriated formulation directly or subjected to a concentration step to reach a more suitable composition. Different protocols can be used to concentrate the cell-free extract, such as dehydration, filtration, ultra-filtration, centrifugation, ultra-centrifugation, precipitation, or chromatography. Dehydration may be carried out through a lyophilisation process, by fluidised bed drying, by spray drying or drying in an oven under vacuum.

A fourth aspect of the invention refers to an inoculation product comprising the strain as defined in the first aspect of the invention.

By "inoculation product" it is understood a product obtained after inoculating the strain in a suitable culture medium, subjecting the inoculated culture medium to suitable growth conditions.

In an embodiment of the fourth aspect of the invention, optionally in combination with any of the embodiments provided above or below, the inoculation product comprises a viable strain as defined in the first aspect of the invention.

In another embodiment of the fourth aspect of the invention, optionally in combination with any of the embodiments provided above or below, the inoculation product comprises the strain of the invention inactivated.

The term "inactivated" means that the micro-organism is not able to form colonies. In one embodiment, the inactivated micro-organisms have the cell membrane intact or broken.

The term "inoculation product comprising the strain of the invention inactivated" refers to a product obtained after inoculating the strain in a suitable culture medium, subjecting the inoculated culture medium to suitable growth conditions, and then inactivating the strain.

With a view to practical use in pest control, pesticide agents are usually formulated into compositions also including agriculturally acceptable compounds. Therefore, a fifth aspect of the present invention refers to a composition comprising the strain as defined in the first aspect or the inoculation product as defined in the fourth aspect of the invention, and one or more agriculturally acceptable compound(s).

The term "effective amount" as used herein, means an amount of the strain of the invention high enough to provide the desired benefit, either the treatment or prevention of the plant disease, but low enough to avoid serious side-effects. The particular dose administered according to this invention will of course be determined by the particular circumstances surrounding the case, including the compound administered, the route of administration, the particular condition being treated, and the similar considerations.

In an embodiment of the fifth aspect of the invention, optionally in combination with any of the embodiments provided above or below, the strain is present at a concentration from $10^5$ cfu/mL to $10^{12}$ cfu/mL. In another embodiment of the fifth aspect of the invention, the strain is present at a concentration of $10^5$ cfu/mL, $10^6$ cfu/mL, $10^7$ cfu/Ml, $10^8$ cfu/mL, $10^9$ cfu/mL, $10^{10}$ cfu/mL, $10^{11}$ cfu/mL or $10^{12}$ cfu/mL.

For the purposes of the invention, any ranges given include both the lower and the upper end-points of the range.

The amount indicated as cfu/mL relates to the colony forming units (CFU) of the strain of the invention per mL.

In another embodiment of the fifth aspect of the invention, optionally in combination with any of the embodiments provided above or below, the composition is a phytosanitary composition.

In another embodiment of the fifth aspect of the invention, optionally in combination with any of the embodiments provided above or below, the agriculturally acceptable compound is selected from the group consisting of: plant strengtheners, nutrients, wetting agents, compounds that improve adherence, buffering compounds, stabilizers, antioxidants, osmotic protectors and sunscreens.

In another embodiment of the fifth aspect of the invention, optionally in combination with any of the embodiments provided above or below, the composition comprises at least one additional pesticide.

In another embodiment of the fifth aspect of the invention, optionally in combination with any of the embodiments provided above or below, the additional pesticide is selected from the group consisting of another bacterial strain with pesticide properties, a fungicide, a bactericide, an herbicide, a chemical insecticide or a chemical nematicide. Said additional pesticide does not have to adversely affect the activity/viability of the strain of the invention included in the composition.

In the present invention, the term "pesticide" is understood by its usual meaning in the field of agronomy as a product intended to kill, repel, regulate or disrupt the growth of living organisms that are considered pests. Clearly, due to the nature of the strain of the invention, herein it is understood that "pesticide" is a biological or ecological (organic) pesticide, also called biopesticide. In the scope of the present invention, the term "pesticide" would have the same meaning as the term "phytosanitary".

In an embodiment of the fifth aspect of the invention, optionally in combination with any of the embodiments provided above or below, it can be in the form of a solution, a pellet, a suspension, a lyophilized compositions or other dried compositions (for example freeze dried composition). The lyophilized or dried composition can be reconstituted with a liquid carrier prior to its use or directly used. The composition may be prepared according to various formulations suitable for phytosanitary uses, for example, chosen from the group consisting of formulations of the following type: liquid intended for use without dilution (AL), powder intended for use without dilution (AP), encapsulated granule (CG), contact liquid or gel (CL), contact powder (CP), powdering powder (DP), emulsifiable concentrate (EC), emulsifiable granule (EG), oil type emulsion (EO), water type emulsion (EW), fine granule (FG), macrogranules (GG), emulsifiable gel (GL), powder for spraying (GP), granules (GR), grease (GS), water-soluble gel (GW), microemulsion (ME), microgranules (MG), water-dilutable concentrated suspension (OF), oil dispersion (OD), water-miscible suspension (OL), powder for dispersion in oil (OP), concentrated in gel or paste form (PC), sticks (for agri-pharmaceutical use) (PR), concentrated suspension (SC), suspoemulsion (SE), water-soluble granules (DG), soluble concentrate (SL), film-forming oil (SO), water-soluble powder (SP), water-soluble tablets (ST), tablets (TB), water-dispersible granules (WG), wettable powder (WP), water-dispersible tablets (WT) (the code consisting of two capital letters corresponding to the international codes for phytosanitary formulations).

In an embodiment of the fifth aspect of the invention, optionally in combination with any of the embodiments provided above or below, the composition is a Technical Grade Active Ingredient (TGAI) composition.

In an embodiment of the fifth aspect of the invention, optionally in combination with any of the embodiments provided above or below, the composition is a lyophilized or dried composition and comprises a concentration of the strain as defined in the first aspect of the invention in the range from $5{,}0 \times 10^{10}$ and $2 \times 10^{11}$ CFU/g or ml.

In an embodiment of the fifth aspect of the invention, optionally in combination with any of the embodiments provided above or below, the composition is a lyophilized or dried composition and comprises a concentration of the strain as defined in the first aspect of the invention of $1{,}0 \times 10^{11}$ CFU/g.

In an embodiment of the fifth aspect of the invention, optionally in combination with any of the embodiments provided above or below, the composition is an oil dispersion (OD) composition.

In an embodiment of the fifth aspect of the invention, optionally in combination with any of the embodiments provided above or below, the composition is an OD formulation and comprises a concentration of the strain as defined in the first aspect of the invention in the range from $1{,}0 \times 10^9$ to $1{,}0 \times 10^{10}$ CFU/mL.

In an embodiment of the fifth aspect of the invention, optionally in combination with any of the embodiments provided above or below, the composition is an OD formulation and comprises a concentration of the strain as defined in the first aspect of the invention of $5 \times 10^9$ CFU/mL.

"Agriculturally acceptable compounds" refers to those compounds and/or materials, which are suitable for use in agriculture. In general, said compounds should be non-toxic to humans and preferably should be environment-friendly.

In an embodiment of the fifth aspect of the invention, the compositions of the invention may contain compounds for improving the adhesion of the strains in the plants to be treated, as well as phytostrengthener compounds, nutrients, wetting agents, stabilizers, osmotic protectors, antioxidants, sunscreens, buffering compounds or combinations thereof.

Examples of adhesion products are gelatin, starch, pectins, alginates and various types of gums such as xanthan. Many of these compounds are also wetting agents. In the case of sunscreens, Congo red, calcium carbonate and wax emulsions can be used. The phytostrengtheners are compounds that can facilitate make crops develop robustness or tolerance towards pathogens or adverse environmental conditions, for example, jasmonic acid analogues and some plant defense stimulants such as harpins, chitosans, and laminarins. Additionally, examples of osmotic protectors are trehalose, betaines and amino acids. Finally, ascorbic acid and glutathione are included among antioxidants.

The compositions of the fifth aspect of the invention can be prepared by routine protocols, such as by mixing the different ingredients.

In view of the above, the seventh aspect of the invention refers to the use of the strain as defined in the first aspect of the invention or the inoculation product as defined in the fourth aspect of the invention, or the composition as defined in the fifth aspect of the invention as a pesticide in plants.

In an embodiment of the seventh aspect of the invention, the strain inoculation or composition is used for controlling a disease caused by a bacterium or fungus in a plant

In the present invention, the term "control of the disease" means that it prevents, reduces, cures or eradicates the disease.

In an embodiment of the seventh aspect of the invention, the strain is used in preventing infections caused by bacteria or fungi in plants.

The eight aspect of the invention refers to a method for controlling an infection caused by bacterial or fungal pathogen in a plant. In one embodiment of said aspect, optionally in combination with any of the embodiments provided above or below, the method comprises applying the strain as defined in the first aspect of the invention to the affected part of the plant and/or to the seed or to the substrate or soil used for growing said plant. In a further embodiment, optionally in combination with any of the embodiments provided above or below, the method comprises applying the inoculation product as defined in the fourth aspect of the invention to the affected part of the plant and/or to the seed or to the substrate or soil used for growing said plant. In an even further embodiment, optionally in combination with any of the embodiments provided above or below, the method comprises applying the composition as defined in the fifth aspect of the invention, to the affected part of the plant and/or to the seed or to the substrate or soil used for growing said plant.

The dose may be adapted according to the composition of the fifth aspect of the invention and the formulation of the composition which is used and also according to the weather conditions, any resistance phenomena or other natural factors, the nature of the treatment or the degree of infestation, and according to the plants or sites to be treated.

In an embodiment of the eight aspect of the invention, optionally in combination with any of the embodiments provided above or below, the strain the inoculation product or the composition is applied to plant nursery boxes, during seed treating, seed dressing, seed disinfection, seedling root dipping treatment, planting pit treatment, plant foot treatment, planting row treatment, surface spraying, soil incorporation, or by application to a water culture medium in hydroponic culture.

In an embodiment of the eighth aspect of the invention, optionally in combination with any of the embodiments provided above or below, the part of the plant treated is the seed. This treatment can be achieved by ordinary methods; for example, a method wherein seeds are dipped in the composition of the fifth aspect of the invention.

The treatment of the plant, i.e. root and/or the seeds, allows the bactericidal and fungicidal action of the strain of the invention.

The term "plant" comprises all plant species cultivated by humans, in particular those intended for food or for animal feed, such as cereals, fodder, vegetable, fruit crops, vines, and/or for the supply of wood for all purposes (such as heating, housing construction furniture, and/or ornamentation). Example of plants include cereals (for example, rice, barley, wheat, rye, oat and corn), beans (for example, soybean, azuki bean, broad bean, peas and peanuts), fruit trees/fruits (for example, apples, pears, citruses, grapes, peaches, apricots, cherries, olive, nuts, almonds, bananas, berries and strawberries), vegetables (for example, tomato, cabbage, spinach, broccoli, lettuce, onion, garlic, leek and pepper), root crops (for example, potato, carrot, sweet potato, radish, lotus root and turnip), industrial crops (for example, cotton, hemp, paper mulberry, mitsumata, rape, beet, hop, sugarcane, sugar beet, rubber, coffee, tobacco, tea), pepos (for example, pumpkin, cucumber, watermelon, melon), pasture plants (for example, orchardgrass, sorghum, Thimothy-grass, clover, alfalfa), lawn grasses (for example, mascarene grass, bentgrass), crops for flavorings (for example, lavender, rosemary, thyme, parsley, basilica, mint, coriander, pepper, ginger), and flower plants (for example, chrysanthemum, rose, orchids).

The term "part of a plant" includes any segment of the plant, such as the root, stem, leaves and seeds.

The term "seed" includes what is called seeds, and also plant bodies for vegetative propagation such as bulbs, tubers and seed potato.

In an embodiment of the seventh or eighth aspects of the invention, optionally in combination with any of the embodiments provided above or below, the plant is tomato, or potato plant.

The term "substrate" comprises any support for cultivating a plant, and the material therefor is not particularly limited, as far as the plant can grow therein; for example, nursery mats, water, sand, soil, vermiculite, cotton, paper, diatomaceous earth, agar, gel substances, polymeric substances, rock wool, glass wool, wood chips, barks and pumice.

In an embodiment of the eighth aspect of the invention, optionally in combination with any of the embodiments provided above or below, the method of application is performed in the vicinity of the plant or a nursery bed for raising seedlings.

In an embodiment of the eighth aspect of the invention, optionally in combination with any of the embodiments provided above or below, the method of application is performed directly in the plant.

The examples below demonstrate the use of a strain of the invention as a bio-pesticide, in the control of bacterial and fungal infections.

In an embodiment of the seventh or eighth aspects of the invention, optionally in combination with any of the embodiments provided above or below, the use of the seventh aspect and the method of the eighth aspect are for controlling the colonization of pest of bacteria and/or fungi or treating infested plants. In particular, the invention relates to uses for controlling those plant pests. The term "treating" therefore is referred to infested plants. The term control comprises preventing infestations of the plants by said pests, repelling or eliminating said pests.

In an embodiment of the seventh or eighth aspects of the invention, optionally in combination with any of the embodiments provided above or below, the use of the seventh aspect and the method of the eighth aspect are for treating a plant infected by pest of bacteria and/or fungi.

In an embodiment of the seventh or eighth aspects of the invention, optionally in combination with any of the embodiments provided above or below, the bacteria are selected from the genus *Xanthomonas, Pseudomonas* and *Erwinia.*

In an embodiment of the seventh or eighth aspects of the invention, optionally in combination with any of the embodiments provided above or below, the bacteria are selected form the group consisting of *Xanthomonas vesicatoria, Pseudomonas syringae, and Erwinia Carotovora.*

In an embodiment of the seventh or eighth aspects of the invention, optionally in combination with any of the embodiments provided above or below, the fungi are selected from the genus *Rhizoctonia* and *Botrytis.*

In an embodiment of the seventh or eighth aspects of the invention, optionally in combination with any of the embodiments provided above or below, the fungi are selected form the group consisting of *Rhizoctonia solani* and *Botrytis aclada.*

In an embodiment of the seventh or eighth aspects of the invention, optionally in combination with any of the embodiments provided above or below, the bacteria are selected from the genus *Xanthomonas, Pseudomonas* and *Erwinia*; and wherein the fungi are of the genus *Rhizoctonia* and *Botrytis.*

In an embodiment of the seventh or eighth aspects of the invention, optionally in combination with any of the embodiments provided above or below, the bacteria is selected from the genus *Xanthomonas, Pseudomonas* and *Erwinia*; or alternatively, it is selected from the group consisting of *Xanthomonas vesicatoria, Pseudomonas syringae, and Erwinia carotovora;* or alternatively, the fungi is of the genus *Rhizoctonia* and *Botrytis;* or, alternatively, it is selected from the group consisting of *Rhizoctonia solani* and *Botrytis aclada;* or, alternatively, the bacteria is selected from the genus *Xanthomonas* and the fungi is of the genus *Rhizoctonia;* or, alternatively, the bacteria is selected from the genus *Xanthomonas* and the fungi is of the genus *Botrytis;* or, alternatively, the bacteria is selected from the genus *Pseudomonas* and the fungi is of the genus *Rhizoctonia;* or, alternatively, the bacteria is selected from the genus *Pseudomonas* and the fungi is of the genus *Botrytis;* or, alternatively, the bacteria is selected from the genus *Erwinia* and the fungi is of the genus *Rhizoctonia;* or, alternatively, the bacteria is selected from the genus *Erwinia* and the fungi is of the genus *Botrytis;* or, alternatively, the bacteria is selected from the genus *Xanthomonas* and the fungi is *Rhizoctonia solani* and *Botrytis aclada;* or, alternatively, the bacteria is selected from the genus *Pseudomonas* and the fungi is *Rhizoctonia solani* or *Botrytis aclada;* or, alternatively, the bacteria is selected from the genus *Erwinia* and the fungi is *Rhizoctonia solani* or *Botrytis aclada;* or, alternatively, the bacteria is *Xanthomonas vesicatoria, Pseudomonas syringae, or Erwinia carotovora* and the fungi is of the genus *Rhizoctonia;* or, alternatively, the bacteria is *Xanthomonas vesicatoria, Pseudomonas syringae, or Erwinia carotovora* and the fungi is of the genus *Botrytis;* or alternatively, the bacteria is selected from the group consisting of *Xanthomonas vesicatoria, Pseudomonas syringae, and Erwinia carotovora;* and the fungi is selected from the group consisting of *Rhizoctonia solani* and *Botrytis aclada;* or alternatively, the bacteria is *Xanthomonas vesicatoria* and the fungi is *Rhizoctonia solani;* or alternatively, the bacteria is *Xanthomonas vesicatoria* and the fungi is *Botrytis aclada;* or alternatively, the bacteria is *Pseudomonas syringae* and the fungi is *Rhizoctonia solani;* or alternatively, the bacteria is *Pseudomonas syringae* and the fungi is *Botrytis aclada;* or alternatively, the bacteria is *Erwinia carotovora* and the fungi is *Rhizoctonia solani;* or alternatively, the bacteria is *Erwinia carotovora* and the fungi is *Botrytis aclada.*

In the present invention the term "infection" comprises asymptomatic infection or symptomatic infection of the plant caused by a nematode.

In an embodiment of the seventh or eighth aspects of the invention, optionally in combination with any of the embodiments provided above or below, the control can be performed in any plant such as potato or tomato plant.

A ninth aspect of the present invention relates to a kit comprising an effective amount of the strain as defined in the first aspect of the invention or the inoculation product as defined in the fourth aspect of the invention, or the composition as defined in the fifth aspect of the invention.

All the embodiments provided above for the strain the inoculation product and the composition are also embodiments of the kit of the ninth aspect of the invention.

In an embodiment of the ninth aspect of the invention, optionally in combination with any of the embodiments provided above or below, the kit may comprise a weekly, monthly, or other periodic dose of the strain of the first aspect of the invention. As an illustrative example, a kit comprising a weekly dose may comprise seven discrete compositions comprising the strain (seven daily doses). As another example, a kit comprising a monthly dose may comprise thirty compositions comprising the strain of the first aspect of the invention.

In case the strain of the first aspect of the invention is lyophilized, the kit of the ninth aspect of the invention can contain an edible resuspension agent, such as water.

In another embodiment of the ninth aspect of the invention, optionally in combination with any of the embodiments provided above or below, the kit comprises means to facilitate dosing compliance. For example, the kits may be particularly advantageous for the purpose of ensuring that the person who used it is performing the correct administration of the effective amount of the strain of the invention to the plant on the appropriately prescribed schedule. Blister cards or other containing devices appropriately configured may be particularly suitable for clearly illustrating sequence or timing of administration of the various components. The kit may be obtained as one card, or cases of four, six, seven (e.g., a weekly supply), or eight cards co-packaged together. Additionally, monthly or other types of kits may be obtained.

The kit that comprises the isolated strain of the first aspect of the invention can comprise any means that allows the correct culture of the strain of the invention, such as culture medium, supplements or antibiotics, as well as instructions for the correct preparation and/or application to the plant.

A tenth aspect of the invention refers to the use of the kit of the ninth aspect of the invention for controlling an infection caused by bacterial pathogens and/or fungal pathogens in a plant.

All the embodiments provided above under the seventh and eighth aspects of the invention, regarding the particular infections and plants, among others, are also embodiments of the ninth and tenth aspects.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples:

### Example 1. Characterization of the *Pseudomonas sp.* strain B2021 (CECT8708)

#### *A. Genomic characterization*

#### *B2021 genome sequencing*

The B2021 whole genome shotgun sequence was obtained using the same methods applied previously for *Lysobacter enzymogenes* (Hernández, I. and C. Fernàndez. 2017. Draft genome sequence and assembly of a *Lysobacter enzymogenes* strain with biological control activity against root knot nematodes. Genome Announc 5(18): e00271-00217), which make use of standard high-throuput sequencing thechnology and bioinformatic analysis tools. In short, genomic DNA was extracted with a commercial kit (Quick-DNA Fungal/Bacterial kit; Zymo Research, Irvine, California, USA) following manufacturer's instructions. The DNA was submitted to a sequencing service provider (Genomix4Life, Baronissi, Italy) for library preparation (Nextera DNA library prep kit, Illumina, Cambridge, UK) and sequencing (2×150 paired-end mode) in a NextSeq500 device (Illumina). All library preparations and sequencing were performed according to supplier's specifications. The raw reads (raw data generated by the sequencing device) were trimmed for quality and length using Trim Galore! v0.4.0 (http://www.bioinformatics.babraham.ac.uk/projects/trim galore/) and the trimmed reads were assembled using SPADes v3.5.0 (Bankevich, A., et al. 2012. SPAdes: a new genome assembly algorithm and its applications to single-cell sequencing. Journal of Computational Biology 19(5): 455-477). The assembled contiguous sequences (contigs) were filtered by their length and depth of coverage so contigs shorter than 200 nt or with coverage below 10-fold were discarded.

### *16s Sequencing*

The 16S rRNA gene sequencing was performed as described next. A PCR reaction mix, which consisted of 10 μL Phire Green Hot Start II Master Mix (Thermo Fisher Scientific), 0.8 μL primer 8f 10 μM (SEQ ID NO:2 5'-AGAGTTTG ATCCTGGCTCAG-3'), 0.8 μL primer 1492r 10 μM (SEQ ID NO:3 5'-ACCTTGTTACGACTT-3'), 2 μL sample DNA and 6.4 μL nanopure water, was amplified according to the following program: 98°C 5 min; 35 cycles of 98°C 5 s, 58.5°C 5 s and 72°C 20 s, and 72°C 20 s. The PCR product was purified using the EZNA Cycle Pure Kit (Omega Bio-Tek) according to manufacturer's instructions. The purified PCR product was sequenced by an external sequencing sercive (Secugen) using the primer 1492r (SEQ ID NO: 2) and the Sanger method with BigDye® Terminator v3.1 (Applied Biosystems) according to manufacturer's instructions.

The homology searches of the 16S gene were carried out using the BLAST suite (12, 13) (https://blast.ncbi.nlm. nih.gov/Blast.cgi?PAGE_TYPE=BlastSearch). The applied parameters were those established by default except for the following:

- Database: Nucleotide collection (nr / nt)

- Organism: Pseudomonas (taxid: 286)

- Limit to: Sequences from type material

- Optimized for: Somewhat similar sequences (blastn)

**Table 1.** Homology searches results of the consensus sequence 16Sof B2021 in NCBI database.

| Hit | Max Score | Total Score | Query cov. | E-val | Ident | Accession |
|---|---|---|---|---|---|---|
| *Pseudomonas azotoformans* strain LMG 21611 genome assembly, chromosome: I | 1822 | 9132 | 100% | 0.0 | 100% | LT629702.1 |
| *Pseudomonas* sp. DSM 29167 16S ribosomal RNA gene, partial sequence | 1822 | 1822 | 100% | 0.0 | 100% | KP756923.1 |
| *Pseudomonas azotoformans* strain NBRC 12693 16S ribosomal RNA gene, partial sequence | 1819 | 1819 | 100% | 0.0 | 99% | NR_113600.1 |
| *Pseudomonas* sp. DSM 29164 16S ribosomal RNA gene, partial sequence | 1817 | 1817 | 100% | 0.0 | 99% | KP756921.1 |
| *Pseudomonas* synxantha strain LMG 2190 genome assembly, chromosome: I | 1808 | 12486 | 100% | 0.0 | 99% | LT629786.1 |
| *Pseudomonas azotoformans* gene for 16S ribosomal RNA, partial sequence, strain: IAM 1603, clone: N3-903 | 1808 | 1808 | 100% | 0.0 | 99% | LC130640.1 |
| *Pseudomonas azotoformans* gene for 16S ribosomal RNA, partial sequence, strain: IAM 1603, clone: C2-114 | 1808 | 1808 | 100% | 0.0 | 99% | LC130639.1 |
| *Pseudomonas mucidolens* strain LMG 2223 genome assembly, chromosome: I | 1804 | 9053 | 100% | 0.0 | 99% | LT629802.1 |
| *Pseudomonas mucidolens* strain NBRC 103159 16S ribosomal RNA gene, partial sequence | 1804 | 1804 | 100% | 0.0 | 99% | NR_114225.1 |

(continued)

| Hit | Max Score | Total Score | Query cov. | E-val | Ident | Accession |
|---|---|---|---|---|---|---|
| *Pseudomonas synxantha* strain NBRC 3913 16S ribosomal RNA gene, partial sequence | 1804 | 1804 | 100% | 0.0 | 99% | NR_113583.1 |

The sequencing revealed a sequence (SEQ ID NO: 4). As shown in Table 1, the sequence was 100% identical to that of *Pseudomonas azotoformans* strain LMG 21611, and to that of a *Pseudomonas sp.* DSM29167 strain, and 99% identical to that of *Pseudomonas azotoformans* strain NBRC12693, *Pseudomonas sp* strain DSM29164, and to that of *Pseudomonas synxantha* strain LMG2190, among others.

**rpoD, gyrA and gyrB Sequencing**

The sequences of the rpoD, gyrA and gyrB genes were obtained from the genome of B2021 using the SEED Viewer browser within the RAST Annotation Server.

The homology searches of the rpoD, gyrA and gyrB genes were carried out as described in 16s sequencing section.

**Table 2.** Homology searches results of the gene sequence rpoD of B2021 (SEQ ID NO: 5) in the NCBI database.

| Hit | Max Score | Total Score | Query cov. | E-val | Ident | Accession |
|---|---|---|---|---|---|---|
| *Pseudomonas fluorescens* strain ATCC 13525 genome assembly, chromosome: I | 419 | 24157 | 100% | 4e-116 | 80% | LT907842.1 |
| *Pseudomonas azotoformans* strain LMG 21611 genome assembly, chromosome: I | 414 | 13610 | 99% | 2e-114 | 79% | LT629702.1 |
| *Pseudomonas yamanorum* strain LMG 27247 genome assembly, chromosome: I | 410 | 39625 | 100% | 2e-113 | 79% | LT629793.1 |
| *Pseudomonas arsenicoxydans* strain CECT 7543 genome assembly, chromosome: I | 161 | 5204 | 90% | 2e-38 | 69% | LT629705.1 |
| *Pseudomonas fragi* strain NRRL B-727 genome assembly, chromosome: I | 118 | 7111 | 95% | 2e-25 | 67% | LT629783.1 |

As shown in table 2, when rpoD gene sequence of the strain of the invention B2021 was searched for homologies in the NCBI database, different species of *Pseudomonas* genus appeared. The ones which presented higher homology were *P. fluorescens* ATCC13525, *P. azotoformans* LMG21611, and the *P. yamanorum* LMG2747.

**Table 3.** Homology searches results of the gene sequence gyrB of B2021 (SEQ ID NO: 6) in the NCBI database.

| Hit | Max Score | Total Score | Query cov. | E=val | Ident | Accession |
|---|---|---|---|---|---|---|
| *Pseudomonas synxantha* strain LMG 2190 genome assembly, chromosome: I | 3752 | 4364 | 100% | 0.0 | 94% | LT629786.1 |
| *Pseudomonas azotoformans* strain LMG 21611 genome assembly, chromosome: I | 3667 | 4117 | 100% | 0.0 | 94% | LT629702.1 |
| *Pseudomonas fluorescens* strain ATCC 13525 genome assembly, chromosome: I | 3517 | 4257 | 100% | 0.0 | 92% | LT907842.1 |

(continued)

| Hit | Max Score | Total Score | Query cov. | E=val | Ident | Accession |
|---|---|---|---|---|---|---|
| *Pseudomonas extremaustralis* strain DSM 17835 genome assembly, chromosome: I | 3508 | 3918 | 100% | 0.0 | 92% | LT629689.1 |
| *Pseudomonas yamanorum* strain LMG 27247 genome assembly, chromosome: I | 3494 | 3946 | 100% | 0.0 | 92% | LT629793.1 |

GyrB gene was also analysed as rpoD and 16S rRNA, in this case, the sequences of this gene which exhibited higher identity to the one from the B2021 strain, belonged to five different species of *Pseudomonas* genus, *P. synxantha* LMG2190, the *P. azotoformans* LMG21611, the *P. fluorescens* ATCC13525, the *P. extremaustralis* DSM 17835, and the *P. yamanorum* LMG27247 (Table 3).

**Table 4.** Homology searches results of the gene sequence gyrA of B2021 (SEQ ID NO: 7) in NCBI database.

| Hit | Max Score | Total Score | Query cov. | E-val | Ident | Accession |
|---|---|---|---|---|---|---|
| *Pseudomonas azotoformans* strain LMG 21611 genome assembly, chromosome: I | 4320 | 5226 | 100% | 0.0 | 96% | LT629702.1 |
| *Pseudomonas synxantha* strain LMG 2190 genome assembly, chromosome: I | 4269 | 5004 | 100% | 0.0 | 96% | LT629786.1 |
| *Pseudomonas extremaustralis* strain DSM 17835 genome assembly, chromosome: I | 4221 | 5372 | 100% | 0.0 | 95% | LT629689.1 |
| *Pseudomonas fluorescens* strain ATCC 13525 genome assembly, chromosome: I | 4114 | 4872 | 100% | 0.0 | 94% | LT907842.1 |
| *Pseudomonas yamanorum* strain LMG 27247 genome assembly, chromosome: I | 4006 | 4805 | 100% | 0.0 | 93% | LT629793.1 |

Finally, the results of the analysis of gyrA gene, went in the same direction, the closest sequences belonged to *P. azotoformans* LMG21611, *P. synxantha* LMG2190, *P. extremaustralis* DSM 17835, *P. fluorescens* ATCC13525, and the *P. yamanorum* LMG27247 (Table 4).

Taking into account the results of this homology analysis, there were three species which were considered to be the more closely related with the strain of the invention that are *P. fluorescens* ATCC13525, the *P. azotoformans* LMG21611, and the *P. yamanorum* LMG2747, as they appeared in the results of all the genes examined in the first five positions.

***Average nucleotide identity (ANI) calculation***

The ANI, both ANIb (based on the BLAST algorithm) and the ANIm (based on the MUMmer algorithm) were calculated on the Jspecies web server (http://jspecies.ribohost.com/jspeciesws).

It should be noted that due to the nature of the algorithms used, the calculation of reciprocal ANIs can give slightly different values. However, these differences are usually inferiors to 0.1%.

The characteristics of the genomes used for the ANI calculation are provided in Table 5.

**Table 5.** Genome characteristics of the genomes compared in the study.

| Genome | Size (bases) | Contigs | GC (%) |
|---|---|---|---|
| B2021 | 6096505 | 124 | 60.3 |
| *Pseudomonas fluorescens* ATCC13525 | 6511547 | 1 | 60.0 |

(continued)

| Genome | Size (bases) | Contigs | GC (%) |
|---|---|---|---|
| *Pseudomonas azotoformans* LMG21611 | 6727386 | 1 | 61.0 |
| *Pseudomonas fluorescens* NCTC10038 | 651517 | 1 | 60.0 |
| *Pseudomonas yamanorum* LMG27247 | 7087525 | 1 | 60.3 |
| *Pseudomonas azotoformans* S4 | 6859618 | 1 | 60.3 |
| *Pseudomonas azotoformans* F77 | 6581295 | 1 | 60.6 |
| Contigs= contiguous sequence embedded from shorter sequences (reads) and which does not contain unknown nucleotides (gaps). A value of "124" means that the genome is fragmented into 124 pieces whose order is unkown. | | | |

For ANIb and ANIm calculations, the parameters used were those appearing by default in JSpecies web server. Results are shown in Tables 6 and 7, below.

As a control, the ANIb and ANIm values were calculated between *Pseudomonas fluorescens* ATCC13525 and *Pseudomonas fluorescens* NCTC10038. The results were ANIb 99.99% and ANIm 99.98% when using NCTC10038 as template and ANIb 100.00% and ANIm 99.98% when using ATCC13525 as the template. These results confirm that these two strains fulfil the criteria for deemed them to be from the same specie according to ANI calculation.

**Table 6.** ANIb values calculated from B2021 and the species more closely related to it.

| Genome | ANIb (%) | Align (%) | Align (bp) | Total (bp) |
|---|---|---|---|---|
| *Pseudomonas azotoformans* S4 | 86.40 | 73.28 | 4467287 | 6096505 |
| *Pseudomonas azotoformans* LMG21611 | 86.24 | 76.341 | 4654172 | 6096505 |
| *Pseudomonas azotoformans* F77 | 86.11 | 74.96 | 4570190 | 6096505 |
| *Pseudomonas fluorescens* ATCC13525 | 85.92 | 74.83 | 4561969 | 6096505 |
| *Pseudomonas yamanorum* LMG27247 | 84.26 | 74.32 | 4530941 | 6096505 |

**Table 7.** ANIm values calculated from B2021 and the species more closely related to it.

| Genome | ANIm (%) | Align (%) | Align (bp) | Total (bp) |
|---|---|---|---|---|
| *Pseudomonas azotoformans* LMG21611 | 88.38 | 72.671 | 4430459 | 6096505 |
| *Pseudomonas azotoformans* S4 | 88.26 | 71.71 | 4372062 | 6096505 |
| *Pseudomonas azotoformans* F77 | 88.31 | 71.54 | 4361686 | 6096505 |
| *Pseudomonas fluorescens* ATCC13525 | 88.08 | 70.70 | 4310388 | 6096505 |
| *Pseudomonas yamanorum* LMG27247 | 87.16 | 66.05 | 4026497 | 6096505 |

As shown in Tables 6 and 7, the highest ANI values between B2021 and any of the strains listed in Table 5 were 86.40% ANIb with *P. azotoformans* S4, and 88.38% ANIm with *P. azotoformans* LMG_21611, however, these values are below the threshold of 94-95% estimated for two strains to be deemed to be from the same species. That is, this indicates that the strain of the invention does not belong to any of the species tested, which were the most closely related according to the 16s rRNA and rpoD, gyrA, and gyrB genes.

**B. Phenotypical characterization**

**MATERIALS AND METHODS**

**Temperature Range**

To determine the optimum temperature for the growth of B2021, the strain was streaked on the surface of Nutrient agar plates that were then incubated at temperatures of 4°C, 18°C, 22°C, 25°C, 28°C, 30°C, 37°C and 40°C. Bacterial growth was evaluated every day during 4 days of incubation.

*pH Range*

pH range of growth at 28°C of B2021 strain was determined in sterile 96-well plates. For the experiment, B2021 was first grown overnight in LB. After that, 100 μL of bacterial suspension with a final optical density at 600 nm (OD600) of 0.01 were added to LB medium at following pHs: 3.46, 3.92, 4.26, 5.08, 7.03, 9.33, 10.04, 10.98. Then, 200 μL of B2021 isolate was inoculated for pH (2 wells per pH). The plates were read after 24h of incubation at 28°C. To determine end points, several approaches were taken into account. Bacteria growth was evaluated by optical density at 620 nm (OD620) and cell viability was confirmed by addition of 30 μL of 0.01% resazurin. Any color changes from blue-purple to pink were recorded as positive.

*Salinity Range*

The effect of salinity on the rate of growth of B2021 strain at 28°C was determined in sterile 96-well. For the experiment, B2021 was first grown overnight in LB. After that, 100 μL of bacterial suspensions with a final optical density at 600 nm (OD600) of 0.01 were added to the wells containing the following NaCl dilutions: 8, 6, 4, 3, 2, 1.5, 1, 0.75, 0.5, 0.375, 0.25 and 0%. The plates were read after 48h of incubation at 28°C. To determine MIC end points, bacteria growth was evaluated by optical density at 620 nm (OD620). After that, cell viability was confirmed by addition of 30 μl of 0.01% resazurin. Any color changes from blue-purple to pink were recorded as positive.

*Antibiotic resistance*

Minimum inhibitory concentrations (MICs) for tested antibiotics were determined by the broth microdilution (BMD) method. Four antibiotics were used: Streptomycine Sulfate, Nalidixic Acid, Oxytetracycline and Erythromycin. Briefly, MICs were determined in sterile 96-well plates by two-fold serial dilutions in 100 μL of LB. B2021 was first grown overnight in LB and, after that, 100 μL of bacterial suspension with a final optical density at 600 nm (OD600) of 0.05 were added to the wells containing the antibiotic dilutions, and the MIC plates were read after 18 h of incubation at 28°C. The MIC was defined as the lowest antibiotic concentration that inhibited 80% of growth (based on OD measurements) in comparison to the control. Moreover, cell viability was confirmed by addition of 30 μL of 0.01% resazurin. Any color changes from blue-purple to pink were recorded as positive and the MIC was defined as the lowest antibiotic concentration lacking this color change.

*Apizym® and Api20NE®*

Enzymatic activities were determined using APIZYM® and API20NE® (bioMérieux, Madrid, Spain), following the manufacturer's instructions.

**RESULTS**

The strain of the invention, B2021, has bacillus shape and Gram Negative Staining. Its pH range for growth is 4-10 and its temperature range for growth is 4-30°C so strain is mesophile and neutrophile (Table 8). The optimal temperature growth range is 28°C-30°C and does not grow at 37°C. B2021 is capable of growing from 0% to 4% of NaCl and it is considered a non halotolerant strain (Table 8). It shows more sensitivity to oxytetracycline and highly resistance to streptomycine sulfate (Table 8).

**Table 8.** Phenotypic characterization of B2021 strain.

|  |  | **B2021** |
|---|---|---|
|  | Temperature range (°C) | 4-30°C |
|  | pH range | 4-10 |
|  | Salinity range (%) | 0-4 |
| Antibiotic resistance (MIC) | Streptomycine Sulfate | 1600 μg/ml |
|  | Nalidixic Acid | 240 μg/ml |
|  | Oxytetracycline | 3,13 μg/ml |
|  | Erythromycine | 240 μg/ml |

(continued)

| | | B2021 |
|---|---|---|
| Enzym production | According to API ZYM | Alkaline phosphatase |
| | | Esterase (C 4) |
| | | Leucine arylamidase |
| | | Acid phosphatase |
| | | Naphtol-AS-BI-phosphohydrolase |
| | According to API 20NE | potassium nitrate |
| | | L-arginine |
| | | gelatin (bovine origin) |
| | | D-glucose |
| | | L-arabinose |
| | | D-mannose |
| | | D-mannitol |
| | | N-acetyl-glucosamine |
| | | potassium gluconate |
| | | capric acid |
| | | malic acid |
| | | trisodium citrate |
| | | oxidase test |
| | Siderophore Production | Positive |
| | Inhibition of Pathogen Biofilm | 58% |

According to APIZYM®, B2021 produces several enzymes like Alkaline phosphatase, Esterase (C4), Leucine arylamidase, Acid phosphatase and, Naphtol-AS-BI-phosphohydrolase (Tables 8 and 9). On the other hand, B2021 strain gives positive results for 13 enzymes of API 20NE®, what means that it may produce them (Tables 8 and 10).

**Table 9.** APYZYM® Results.

| Well | Enzime assayed for | Substrate | Intensity value | Result |
|------|--------------------|-----------|-----------------|--------|
| 1 | Control | - | 0 | - |
| 2 | Alkaline phosphatase | 2-naphthyl phosphate | 4 | + |
| 3 | Esterase (C 4) | 2-naphthyl butyrate | 3 | + |
| 4 | Esterase Lipase (C 8) | 2-naphthyl caprylate | 1 | - |
| 5 | Lipase (C 14) | 2-naphthyl myristate | 0 | - |
| 6 | Leucine arylamidase | L-leucyl-2-naphthylamide | 4 | + |
| 7 | Valine arylamidase | L-valyl-2-naphtylamide | 0 | - |
| 8 | Cystine arylamidase | L-cystyl-2-naphtylamide | 0 | - |
| 9 | Trypsin | N-benzoyl-DL-arginine-2-naphtylamide | 0 | - |
| 10 | α-chymotrypsin | N-glutaryl-phenylalanine-2-naphtylamide | 0 | - |
| 11 | Acid phosphatase | 2-naphtyl phosphate | 3 | + |
| 12 | Naphtol-AS-Bl-phosphohydrolase | Naphtol-AS-Bl-phosphate | 4 | + |
| 13 | α-galactosidase | 6-Br-2-naphthyl-αD-glucopyranoside | 0 | - |
| 14 | β galactosidase | 2-naphtyl-βD-glucopyranoside | 0 | - |
| 15 | β-glucuronidase | Naphthol-AS-Bl-βD-glucuronide | 0 | - |
| 16 | α-glucosidase | 2-naphtyl-αD-glucopyranoside | 0 | - |
| 17 | β-glucosidase | 6-Br-2-naphthyl-βD-glucopyranoside | 0 | - |
| 18 | N-acetyl-β-glucosaminidase | 1-naphthyl-N-acetil-βD-glucosaminido | 0 | - |
| 19 | α-mannosidase | 6-Br-2-naftil-αD-mannopiranosido | 0 | - |
| 20 | α-fucosidase | 2-naftil-αL-fucopiranosido | 0 | - |

**Table 10.** APY20NE® Results.

| Well | Test | Active ingredients | Reactions/Enzymes | Result |
|---|---|---|---|---|
| 1 | NO3 | potassium nitrate | reduction of nitrates to nitrites | + |
| 2 | TRP | L-tryptophane | indole production (TRyptoPhane) | - |
| 3 | GLU-Ferm | D-glucose | fermentation (GLUcose) | - |
| 4 | ADH (Arg) | L-arginine | Arginine DiHydrolase | + |
| 5 | URE | urea | UREase | - |
| 6 | ESC | esculin ferric citrate | hydrolysis ( E -glucosidase) (ESCulin) | - |
| 7 | GEL | gelatin (bovine origin) | hydrolysis (protease) (GELatin) | + |
| 8 | PNPG | 4-nitrophenyl- E D-galactopyranoside | E -galactosidase (Para-NitroPhenyl-ßD-Galactopyranosidase) | - |
| 9 | GLU_Assim | D-glucose | assimilation (GLUcose) | + |
| 10 | ARA | L-arabinose | assimilation (ARAbinose) | + |
| 11 | MNE | D-mannose | assimilation (ManNosE) | + |
| 12 | MAN | D-mannitol | assimilation (MANnitol) | + |
| 13 | NAG | N-acetyl-glucosamine | assimilation (N-Acetyl-Glucosamine) | + |
| 14 | MAL | D-maltose | assimilation (MALtose) | - |
| 15 | GNT | potassium gluconate | assimilation (potassium GlucoNate) | + |
| 16 | CAP | capric acid | assimilation (CAPric acid) | + |
| 17 | ADI | adipic acid | assimilation (ADIpic acid) | - |
| 18 | MLT | malic acid | assimilation (MaLaTe) | + |
| 19 | CIT | trisodium citrate | assimilation (trisodium CITrate) | + |
| 20 | PAC | phenylacetic acid | assimilation (PhenylACetic acid) | - |
| 21 | OX | oxidase test | cytochrome oxidase | + |

**Example 2: *Siderophores production* of *the strain* of *the invention*.**

**MATERIALS AND METHODS**

Production of ferric ion chelates was detected using Chrome Azurol S dye (CAS) methodology (Schwyn B. and Neilands J. B. "Universal chemical assay for the detection and determination of siderophores." Anal Biochem, (1987) 160(1): 47-56) with some modifications. CAS solution was made by dissolving 60.5 mg CAS powder (Sigma-Aldrich) in 50 mL distilled water and 10 mL of Fe(III) solution (1 mM $FeCl_3 \cdot 6H_2O$, 10Mm HCl) were added. Under stirring this solution was slowly mixed with 72.9 mg hexadecyltrimethylammonium bromide (HDTMA, Sigma-Aldrich), dissolved in 40 mL water and the resultant dark blue solution was autoclaved to sterilize and stored in the dark.

To determine siderophore production, overnight bacterial cultures were grown at 28°C for 24h on King B plates (20 g/L casein peptone, 1.5 g/L K2HPO4, 1.5 g/L $MgSO_4 \cdot 7H_2O$, 10 ml glycerol, and 15 g/L agar) containing 1/10 of CAS solution previously prepared.

## RESULTS

Siderophores, small extracellular compounds that form complexes with available iron, are involved in biocontrol activity. Siderophores are able to sequester the limited iron pool in the rhizosphere competing with phytopathogens which also require iron to grow. Following CAS assay in agar media, the inventors determined that B2021 was able to produce siderophores. CAS assays showed the change of dark blue to orange color after incubation with this bacterium, while no change was observed after incubation with other bacteria that do not produced siderophores.

***Example 3. Characterization of cell-free culture supernatants (CS) obtained from the strain** of **the invention.***

*A. Absorbance and HPLC analysis*

**MATERIALS AND METHODS**

Since the most closely related specie to the strain of the invention, according to the genomic analysis, was the *Pseudomonas azotoformans,* the characterization of the cell-free culture supernatant (CS) obtained from B2021 strain was performed comparing it to the supernatant obtained by the collection of *Pseudomonas azotoformans* strain DSM 18862. The characterization was focused on production of siderophores and other metabolites.

Both strains were grown in liquid culture medium KingBmod with lower content of iron (5 g/l acid casein peptone, 1.2 g/L $K_2HPO_4$, 0.25 g/L $MgSO_4 \cdot 7H_2O$, 0.03 g/L $ZnSO_4 \cdot 7H_2O$) or in the same medium supplemented with 200 $\mu$M FeCl3. After incubation in darkness at 28°C 170-200rpm for 1 day, cell-free culture supernatants (CS) were obtained by centrifugation at 4000 g and filtration through a 0.22 $\mu$m pore size filter. To confirm that the supernatants were free of bacterial cells, aliquots of 100 $\mu$L were plated in nutritive agar (AN) and absence of growth was confirmed after incubation at 26°C for 2 days.

The siderophore production in CS was visualized by fluorescence and their UV spectra were measured in a micro-volume spectrophotometer (DS-11 FX, Denovix). CS samples were also analysed by HPLC (eAlliance system, Waters, Milford, MA). Chromatograms were performed using an XBridge C18 reversed-phase column (5$\mu$m 4.6x150mm, Waters) eluted at 1 mL/min with a mobile phase mixture of solvents A [H2O + 0.05% trifluoracetic acid (TFA)] and B (CH3CN + 0.05% TFA). The elution gradient was 0-10%B 10min, 10-40%B 10min, 40-100%B 3min, 100%B 2min, 0%B 5min. Siderophores and other metabolites were detected by UV spectroscopy with a photodiode array detector and chromatograms were obtained by processing at 254nm.

## RESULTS

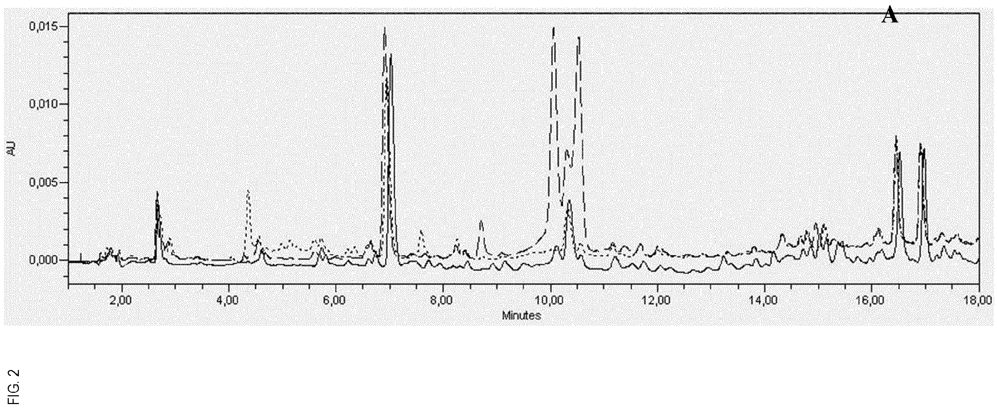

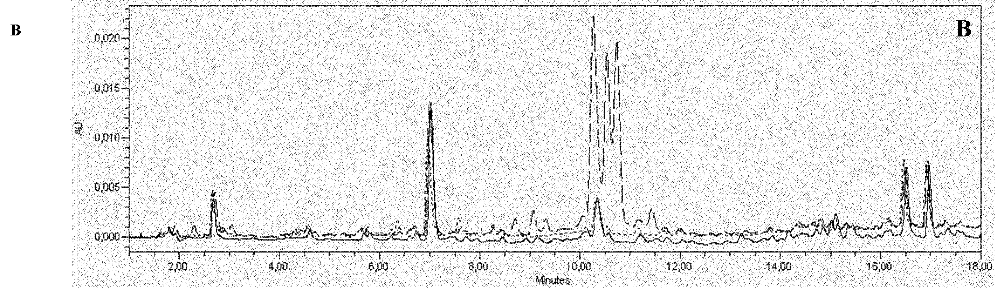

Both cultures obtained from B2021 and DSM 18862 were fluorescent and had a peak of absorbance at 400-420nm indicating the production of fluorescent siderophores, such as pyoverdine-type siderophores produced by *Pseudomonas spp.* These compounds were not produced when the culture medium was supplemented with iron. The peaks corresponding to siderophores and other metabolites were characterized by HPLC (FIG. 2). Siderophore peaks were only observed after incubation of bacteria in medium without iron.

*B. Surfactant activity*

**MATERIALS AND METHODS**

Cell-free culture supernatants were obtained as described in the former section. The surfactant activity or the ability to collapse a droplet of water was tested as described by Kuiper et al (Kuiper I. et al., "Characterization of two Pseudomonas putida lipopeptide biosurfactants, putisolvin I and II, which inhibit biofilm formation and break down existing biofilms". Mol Microbiol, 2004, 51: 97-113). Briefly, 20 $\mu$L of cell-free culture supernatants were pipetted as a droplet onto parafilm. Subsequently, 3 $\mu$L of the dye Congo Red (which had no influence on the shape of the droplets) was added to stain the water and supernatants for photographic purposes. The spreading of the droplet on the parafilm surface was followed over hours until the droplet became dry and the diameter of the dried droplet was recorded.

## RESULTS

As shown in table 11, only the CS obtained from B2021 displayed surfactant activity.

Table 11. Surfactant activity of CS of B2021 and DSM18862 strains.

| Samples | Area (mm$^2$) |
|---|---|
| H$_2$O | 7.1 ± 0.0 |
| KingBmod (control medium) | 2.0 ± 1.7 |
| B2021_KingBmod Bach 1 | 38.5 ± 0.0 |
| B2021_KingBmod Bach 2 | 33.4 ± 7.2 |
| DSM18862_KingBmod Bach 1 | 7.1 ± 0.0 |
| DSM18862_KingBmod Bach 2 | 3.1 ± 0.0 |

**Example 4: Inhibitory activity of the strain of the invention.**

*A. Inhibition of pathogen biofilm of Xanthomonas vesictoria*

**MATERIALS AND METHODS**

Inhibition of biofilm production of DSM 50861 *Xanthomonas vesicatoria* was determined in sterile 96-well plates at 28°C. For the experiment, *X. vesicatoria* and B2021 were first grown overnight in LB at 28°C on a rotary shaker. Next day, *X. vesicatoria* culture was diluted into fresh LB to give an OD600 of 0.1. Sterile untreated 96-well microtiter plates were inoculated with 100 µL of *X. vesicatoria* suspension and 100 µL of free cell supernatant of B2021 which was obtained as provided in Example 3 above, but using LB medium instead of KingBmod. Thus, briefly, the free cell supernatant was obtained with a O/N culture comprising LB, at 28°C and 200 rpm; the supernatant was centrifuged at 5000 g and filtered through a 0.22 µm pore size filter. As a control, 100 µL of supernatant of *X. vesicatoria* were added to the wells without B2021 supernatant. The plates were read after 72h of incubation at 28°C. After incubation optical density of plates were read at 620nm using a plate reader (Multilabel Plater Reader HALO LED96, Dynamica). After that, biofilm were stained with crystal violet 0.1% and diluted with ethanol in order to obtain its optical density at 620 nm. Biofilm production was reported as Relative Biofilm that is the relation of OD of biofilm staining with crystal violet at 620 nm divided by OD at 620 nm of initial cell growth. The results were expressed as the percentage of inhibition compared with the negative control.

**RESULTS**

Microbial biofilm is an aggregate of cells that have organized tissue behavior. Biofilm begins with cell adhesion on a living or inert surface such as a catheter or xylem or phloem from vegetables. It is an important characteristic during microbial colonization and survival in unfavorable environments. Phytopathogen bacteria use to be high biofilm producers because they need it to adhere to the plant to colonize and infect it.
As mentioned above, the strain DSM 50861 *Xanthomonas vesicatoria* was incubated with B2021 supernatant and its own supernatant being the negative control. Results showed that *X. vesicatoria* in presence of its own supernatant may produce biofilm but the presence of B2021 supernatant inhibits biofilm formation (Table 8 and FIG. 1). Supernatant of B2021 strain causes 58.13% of biofilm formation inhibition of *X. vesicatoria* biofilm formation, that is, it could prevent plant infections.

*B. Antifungal activity against* <u>*Rhizoctonia solani*</u>

**MATERIAL AND METHODS**

Two bioassays were performed to assess the biological control activity of B2021 on *Rhizoctonia solani* in potato plants. Both trials were carried on 28 days-old plants in a walk-in growth chamber (Fitoclima 20000®, ARALAB) set at 20/18°C (day/night), 60-70% relative humidity, and a photoperiod of 13/11 h (day/night) with ca. 300 µmol·m$^{-2}$·s$^{-1}$ of photosynthetically-active radiation.
Potato (*Solanum tuberosum*) tubers cultivar "Red Pontiac" were forced to sprout for a period of 2-4 weeks in the dark and then disinfected in a solution of 2% commercial bleach for 10 min, washed with 70% ethanol, and rinsed with sterile tap water. After wiping, potatoes were planted in 1-L containers with sterile substrate (sand:peat:perlite; 4:2:1; v:v:v) and let grow in the growth chamber for 4 weeks. There were 4 different treatments in each bioassay:

1) *Negative Control*: 10 plants without pathogen (30 mL of sterile water was applied to each plant and application time)

2) *Positive Control:* 10 plants inoculated with *R. solani* strain DSM 63010 by applying 30 mL·plant- of a suspension with the pathogen to the substrate at the shoot base. The pathogen solution (0.5 L) was prepared from 4 plates sown with *R. solani* in PDA and grown for 2 weeks at 26 °C and used for treatments 2, 3 and 4.

3) *Biological Control:* Plants inoculated with the pathogen (*R. solani* strain DSM 63010) as before and treated 3 times (days 2, 9, and 15 after transplant) with Actinovate® SP [active ingredient: $1x10^7$ CFU/g *Streptomyces lydicus* strain WYEC108 0.0371% (w:v)] applied at 0.6%-1.0% following manufacturer's instructions (Novozymes) (Table 5)

4) *B2021 treatment*: Plants inoculated with the pathogen (*R. solani* strain DSM 63010) as before and treated with strain B2021 "Technical Grade Active Ingredient" (consisting of freeze dried material composed of 30 % w/w of viable cells and 70% w/w sucrose; TGAI) 2 times (days 2 and 15 after transplant) in Bioassay 1.1. or 3 times (days 2, 9, and 15) in Bioassay 1.2.

Table 12. Experimental design of two *in vivo* growth chamber bioassays to assess efficacy of B2021-TGAI to control *Rhizoctonia solani* in potato plants.

| | | Day 0 | Day 2 | Day 9 | Day 15 | Day 23 |
|---|---|---|---|---|---|---|
| Bioassay | Treatment | Pathogen inoculation* | 1st Application (curative) | 2nd Application (curative) | 3nd Application (curative) | End of the trial |
| 1.1. | B2021-TGAI | √ | $2.70 \ 10^7$ | - | $1.12 \ 10^7$ | √ |
| 1.2. | B2021-TGAI | √ | $3.70 \ 10^6$ | $3.20 \ 10^6$ | $1.25 \ 10^6$ | √ |
| *at trasplant | | | | | | |

Twenty-three days after transplantation the plants were harvested, rinsed with tap water and scored for disease incidence (percentage of damaged plants per treatment) and severity (percentage of stem canker). After verifying data homocedasticity and normality, ANOVA and LSD posthoc tests were performed. Differences were considered statistically significant at $P < 0.05$. All statistical analyses were performed with R.

## RESULTS

The potato plants inoculated with the fungus *Rhizoctonia solani* and treated 2-3 times with B2021-TGAI showed a significant reduction in the percentage of severity of the disease compared with the positive control and the biological control (Table 13 below). The results obtained in these bioassays showed that B2021-TGAI have a fungicidal activity. As shown in table G, the effect of the treatment with the strain of the invention was even more effective than the positive biological control used in the assays.

Table 13. Efficacy on the percentage of severity B2021-TGAI on potato inoculated plants with *Rhizoctonia solani* compared with a negative control (plants without pathogen), a positive control (plants inoculated with pathogen), and a biological control (plants inoculated with the pathogen and treated with a commercial product) in two *in vivo* growth chamber bioassays.

| Bioassay | Treatments | Number of applications | Average 3 applications (CFU/mL) | Percentage of Severity (%) | Efficacy on Percentage of Severity (%) |
|---|---|---|---|---|---|
| 1.1. | Negative Control Positive Control Biological Control* B2021-TGAI | 3 (A, B, C) 2 (B, C) | - - $0.6x \ 10^5$ $1.9 \times 10^7$ | 0 64 a 46 ab 33 b | - - 28 b 48 a |
| 1.2. | Negative Control Positive Control Biological Control* | 3 (A, B, C) | - - $1.0 \times 10^5$ | 0 c 47 a 23 b | - - 51.06 a |

(continued)

| Bioassay | Treatments | Number of applications | Average 3 applications (CFU/mL) | Percentage of Severity (%) | Efficacy on Percentage of Severity (%) |
|---|---|---|---|---|---|
| | B2021-TGAI | 3 (A, B, C) | $1.9 \times 10^7$ | 18 b | 61.70 b |
| *Biological Control: ACTINOVATE applied 3 times (A, B, C) at 0.6% or 1% (commercial dose) | | | | | |

## C. Antibacterial activity against Erwinia carotovora

### MATERIAL AND METHODS

Four bioassays were performed to assess the biological control activity of strain B2021 against *Erwinia carotovora* on potato plants. All efficacy bioassays were performed in a walk-in growth chamber (Fitoclima 20000®, ARALAB) set up with the same conditions than in Example 4B.

Potato (*S. tuberosum*) tubers cultivar " Kennebec" were induced to sprout and disinfected as described for the potato-*R. solani* pathosystem (Example 4B). The most healthy sprouts were planted in peat:vermiculite (1:1; v:v) and let grow for 4 weeks in the growth chamber. Then, the plantlets were transplanted to 1-L pots with soil:peat:perlite (2:1:0.5; v:v:v). There were 2 different treatments in each bioassay (since no effective chemical control treatment is available for this bacterial disease, no chemical treatment was included in this pathosystem) with the following design (Table 14): *Biossay 2.1. and 2.2.*

1) *Positive control*: 8-12 plants were challenged with the pathogen (*E. carotovora* sbsp. *atroseptica* strain DSM 34184) one day after transplantation by adding 20 μL of the pathogen bacterial suspension, at $10^8$ CFU·mL$^{-1}$ from an overnight culture in LB medium at 28 °C, into an artificial wound at the base of the shoot.

2) *B2021-TGAI:* 8-12 plants were inoculated with the pathogen (*E. carotovora* strain DSM 34184) as in the positive control, and treated with strain B2021-TGAI 3 times (day 0, 7, 14) as depicted in table 6, into the soil at the base of the shoot.

*Biossay 2.3. and 2.4.*

1) *Positive control:* 8-12 plants were challenged with the pathogen as in the positive control of 2.1 and 2.2 bioassays.

2) *B2021-OD:* 8-12 plants were inoculated with the pathogen (*E. carotovora* strain DSM 34184) as with the positive control and treated with strain B2021 formulated as "Oil Dispersion" (B2021-OD) 3 times (day 0, 7, 14), into the soil at the base of the shoot (the formulation consisted of: 100g TGAI suspended in a vegetable oil used as carrier (790 g soybean oil) and supplemented with surfactants (75 g alcohol, C12-13, ethoxylated and 25 g glycerol mono oleate) and anticaking agent (10 g silicon)).

**Table 14.** Experimental design of four *in vivo* growth chamber bioassays to assess efficacy of B2021-TGAI or B2021-OD to control *Erwinia carotovora* in potato plants.

| | | Day 0 | Day 6 | Day 7 | Day 14 | Day 21 |
|---|---|---|---|---|---|---|
| Bioassay | Treatment | 1st Application (preventive) (CFU/mL) | Pathogen Inoculation | 2nd Application (curative) (CFU/mL) | 3rd Application (curative) (CFU/mL) | End of the trial |
| 2.1. | B2021-TGAI | $1.30\ 10^8$ | √ | $8.30\ 10^7$ | $1.22\ 10^8$ | √ |
| 2.2. | B2021-TGAI | $3.50\ 10^6$ | √ | $5.00\ 10^7$ | $6.10\ 10^6$ | √ |
| 2.3. | B2021-OD | $4.20\ 10^7$ | √ | $4.20\ 10^7$ | $6.60\ 10^7$ | √ |
| 2.4. | B2021-OD | $5.70\ 10^7$ | √ | $4.20\ 10^7$ | $5.90\ 10^7$ | √ |

Twenty-one days after transplantation plants were scored for bacterial soft rot severity (expressed as percentage of necrotic vascular lesion). After verifying data homocedasticity and normality, ANOVA and LSD posthoc tests were performed. Differences were considered statistically significant at $P < 0.05$. All statistical analyses were performed with R.

## RESULTS

The potato plants inoculated with the bacterium *Erwinia carotovora* and treated 3 times with B2021-TGAI or B2021-OD formulation showed a significant reduction in the percentage of severity of the disease compared with the positive control (Table 15 below). The results obtained in these bioassays showed that the strain of the invention have bactericidal effect when presented in all the formulation tested.

**Table 15.** Efficacy on the percentage of severity of *P. azotoformans* B2021-TGAI and B2021-OD Formulation, on potato plants inoculated with *Erwinia amylovora* compared with a positive control (plants inoculated with the pathogen) in four *in vivo* growth chamber bioassays.

| Bioassay | Treatments | Average 3 applications (CFU/mL) | Percentage of Severity (%) | Efficacy on Percentage of Severity (%) |
|---|---|---|---|---|
| 2.1. | Positive Control<br>B2021-TGAI | -<br>$1.12 \cdot 10^8$ | 38.75 a<br>17.41 b | -<br>55.11 |
| 2.2. | Positive Control<br>B2021-TGAI | -<br>$1.99 \cdot 10^7$ | 57.13 a<br>23.88 b | -<br>58.21 |
| 2.3. | Positive Control<br>B2021-OD | -<br>$5.00 \cdot 10^7$ | 44.82 a<br>7.91 b | -<br>82.58 |
| 2.4. | Positive Control<br>B2021-OD | -<br>$5.27 \cdot 10^7$ | 47.59 a<br>24.08 a | -<br>49.00 |

### D. Antibacterial activity against <u>Pseudomonas syringae</u>

### MATERIAL AND METHODS

Two bioassays were performed to assess the biological control activity of strain B2021 against *Pseudomonas syringae pv tomato* on tomato plants. All efficacy bioassays were performed on 21 days old plants performed in a walk-in growth chamber (Fitoclima 20000®, ARALAB) set up with the same conditions than in Example 4B.

Tomato (*S. lycopersicum*) cultivar "Cuarenteno" plantlets were transplanted to 1-L pots with soil:peat:perlite (2:1:0.5; v:v:v). Then, plants were inoculated with O/N culture of *Pseudomonas syringae pv tomato* B2168 (DSM 50315) grown on King B (10E+08 UFC/mL). Leaves were sprayed until run-off and covered with a plastic bag. After 48h inoculation plants were treated with B2021 and let grow in the growth chamber for 1 week. There were 4 different treatments in each bioassay:

1) *Positive Control (Bioassays 3.1 and 3.2):* 9-10 plants were inoculated with *Pseudomonas syringae* strain DSM 50315 by applying 10 mL·of a suspension with the pathogen to the leaves. The pathogen solution was prepared from O/N culture on king B medium at 28°C and used for treatments 1, 2, 3 and 4.

2) *Chemical Control (Bioassays 3.1 and 3.2):* Plants were inoculated with the pathogen (*Pseudomonas syringae* strain DSM 50315), as in the positive control, and treated once (2 days after transplant) with Cuproflow® (Dicopper chloride trihydroxide 38% [SC] P/V) applied at 0.3% following manufacturer's instructions (Isagro S.P.A)

3) *B2021 treatment*: Plants were inoculated with the pathogen (*Pseudomonas syringae* strain DSM 50315) and treated with strain B2021 TGAI (*Bioassay 3.1*) or B2021-OD once, 2 days after transplant (*Bioassays 3.1 and 3.2*).

**Table 16.** Experimental design of two *in vivo* growth chamber bioassays to assess efficacy of B2021-TGAI and B2021-OD to control *Pseudomonas syringae* in tomato plants.

| | | Day 0 | Day 2 | Day 9 |
|---|---|---|---|---|
| Bioassay | Treatment | Pathogen Inoculation* | 1st Application (curative) (CFU/mL) | End of the trial |
| 3.1. | B2021-TGAI | √ | 1.2E+08 | √ |

(continued)

| | | Day 0 | Day 2 | Day 9 |
|---|---|---|---|---|
| Bioassay | Treatment | Pathogen Inoculation* | 1st Application (curative) (CFU/mL) | End of the trial |
| 3.1. | B2021- OD | √ | 2.0E+08 | √ |
| 3.2. | B2021- OD | √ | 3.9E+07 | √ |
| *At transplant | | | | |

Nine days after transplantation plants were scored for bacterial speck disease of tomato severity (expressed as percentage leaf affected). After verifying data homocedasticity and normality, ANOVA and LSD posthoc tests were performed. Differences were considered statistically significant at $P < 0.05$. All statistical analyses were performed with R.

**RESULTS**

The tomato plants inoculated with the bacterium *Pseudomonas syrinagae* and treated once with B2021-TGAI or B2021-OD formulation showed a significant reduction in the percentage of severity of the disease compared with the positive control and the chemical reference (Table 17 below). The results obtained in these bioassays showed that B2021-TGAI and B2021-OD have a bactericidal activity.

**Table 17.** Efficacy on the percentage of severity of B2021-TGAI and B2021-OD, on tomato plants inoculated with *Pseudomonas syringae* compared with a positive control (plants inoculated with the pathogen) and a chemical control (plants inoculated with the pathogen and treated with a commercial chemical product) in two *in vivo* growth chamber bioassays.

| Bioassay | Treatments | Application (CFU/mL) | Percentage of Severity (%) | Efficacy on Percentage of Severity (%) |
|---|---|---|---|---|
| 3.1. | Positive Control | | 16.7 | |
| | Chemical Control* | | 9 | 46% |
| | B2021-TGAI | 1.2E+08 | 9.3 | 44% |
| | B2021-OD | 2.0E+08 | 11.5 | 31% |
| 3.2. | Positive Control | | 11.4 | |
| | Chemical Control* | | 2.3 | 79% |
| | B2021-OD | 3.9E+07 | 6.6 | 42% |
| *Cuproflow 0.3%( Dicopper chloride trihydroxide 38%) | | | | |

**E. Antimicrobial activity of the cell free extract of the strain of the invention.**

**MATERIALS & METHODS**

Antibacterial activity of the cell free extract obtained from the strain of the invention was evaluated against the bacteria *Pseudomonas syringae* strain B2168 (DSM50315), and *Erwinia carotovora* strain B2169 (DSM 30184) and using a microtiter plate assay (López-García B. et al., "Identification of Novel Hexapeptides Bioactive against Phytopathogenic Fungi through Screening of a Synthetic Peptide Combinatorial Library". Appl Environ Microbiol, 2002, 68: 2453-2460). Pathogenic bacteria were overnight cultured in LB medium at 28°C and 200rpm. The absorbance of the stationary cultures was measured and adjusted to 0.01 with LB medium. The assay mixture contained 100 μl of bacteria suspension at 0.01 of absorbance and 100 μl of CS obtained from the strain of the invention as described in example 3A (tests), or $H_2O$ as control. Plates were incubated at 26°C for 1 day and bacterial growth was determined by measuring optical density at 600nm (OD600) in a HALO LED96 microplate reader (Dynamica). The percentage of growth inhibition was calculated as follow:

$$\% \text{ inhibition}=100-((\text{Abs test})/(\text{Abs control}) \times 100).$$

Antifungal activity of the cell free extract obtained from the strain of the invention was evaluated against the fungus *Sclerotinia sclerotiorum* strain H886 (which forms part of Futureco Bioscience collection) by the Poisoned Food technique. A solution of the CS obtained by strain B2021 as described in example 3A, was pipetted aseptically into temperate PDA to reach a final concentration of 10%. Then, the medium was poured into 9 cm Petri plates and, after solidification, agar discs (6 mm diameter) containing mycelium from the active growing edge of 7 days-old cultures of the pathogenic strain were placed on the center of each plate. Each treatment was done by triplicate. Plates were incubated at 26°C in the dark and the radial mycelial growth of fungal colony was followed by measurement of the shorter and longer radius at different days. The *in vitro* fungal growth on PDA plates poisoned with the CS produced by B2021 was compared with the fungal growth on control plates (PDA alone).

**RESULTS**

As shown in table 18, cell-free supernatant of the strain of the invention showed antimicrobial activity. Moreover, CS obtained by bacteria incubated in culture medium with iron showed lower or no inhibition, that is, their antagonistic activity is partially due to the siderophores, which they produce and secrete.

**Table 18.** Antimicrobial activity of CS of B2021 against the phytopathogenic fungus *Sclerotinia sclerotiorum,* and the bacteria *Pseudomonas syringae,* and *Erwinia carotovora.*

| Phytopathogens | % inhibition | |
|---|---|---|
| | **KingBmod** | **KingBmod + Fe** |
| *Sclerotinia sclerotiorum* | 41.3 ± 7.9 | 5.4 ± 7.7 |
| *Pseudomonas syringe* | 69.0 ± 0.9 | 19.5 ± 2.4 |
| *Erwinia carotovora* | 44.0 ± 0.7 | 5.6 ± 4.6 |

*F. Antifungal activity comparative assay*

**MATERIALS & METHODS**

Fungal inhibition tests were performed against the phytopathogen *Botrytis aclada* strain H16 (CECT2851) by the Poisoned Food technique. A solution of the CS obtained by strain B2021 or the *Pseudomonas azotoformans strain* DSM 18862 was pipetted aseptically into temperate PDA to reach two final concentrations, 10% and 50%. Then, the medium was poured into 9 cm Petri plates and, after solidification, agar discs (6 mm diameter) containing mycelium from the active growing edge of 7 days-old cultures of the pathogenic strain were placed on the center of each plate. Each treatment was done by triplicate. Plates were incubated at 26°C in the dark and the radial mycelial growth of fungal colony was followed by measurement of the shorter and longer radius at different days. The *in vitro* fungal growth on PDA plates poisoned with the CS produced by B2021 or DSM 18862 was compared with the fungal growth on control plates (PDA alone).

**RESULTS**

The inventors found that both cell-free supernatants obtained from B2021 and DSM 18862 showed antifungal activity. Moreover, as described in Example 4E, the antagonistic activity was dependent on the siderophore production capacity by the strain of the invention and DSM strain (Table 19).

Remarkably, the CS obtained from the strain of the invention cultured in a media containing Iron (B2021_KingBmod + Fe), although showing a lower effect, had also antifungal activity, suggesting that not only the siderophores produced are the responsible for the antagonistic activity. This effect is more relevant at higher doses of CS (i.e. 50%)

Moreover, the CS obtained from the strain of the invention, B2021, had higher antifungal activity compared with the collection bacterium DSM 18862. This higher effect may be explained by the production of other metabolites by B2021 strain, such as the ones that confers to the CS the surfactant activity (Table 11 and FIG. 3).

**Table 19.** Antifungal activity of CS of B2021 and DSM18862 strains against the phytopathogenic fungus *Botrytis aclada*.

| Cell-free culture supernatants (CS) | % inhibition | |
|---|---|---|
| | 10% | 50% |
| B2021_KingBmod | 32.1 ± 9.1 | 94.5 ± 0.9 |
| B2021_KingBmod+Fe | 11.4 ± 3.7 | 77.2 ± 3.8 |
| DSM18862_KingBmod | 27.0 ± 1.9 | 88.7 ± 26.6 |
| DSM18862_KingBmod+Fe | 1.3 ± 2.2 | 77.1 ± 4.4 |

**Example 5. Industrial scale production of a composition of the invention.**

For industrial scale production of compositions with the strain of the invention, optimal fermentation conditions described below using a 14L bioreactor in liquid medium can be used, although techniques based on solid media fermentation may also be used, all of them being standard methods used in the microbiological industry.

In order to determine the optimal conditions, a 14L Eppendorf bioreactor was used with 10L GYM medium at 30°C, pH 7,0, pO2 >50% ramp, and stirring at 200- 400 rpm. An exponential culture of the *Pseudomonas sp.* CECT8708 was inoculated at 5% v/v and the operational parameters of the bioreactor were monitored, the growth ending in steady phase in 8 h and getting cellular concentrations of $2.0 \times 10^9$ CFU/mL. Then, the culture was centrifuged in Hanil Biomed /J1250 centrifuge at 5000 g and cells were collected aseptically. At the end of this step, the biomass was concentrated by resuspending in 500 mL buffer, obtaining about 1 litres of $4 \times 10^{10}$ CFU/mL concentrate. An inert osmotic protector ingredient (1L) was added later to the concentrate and dehydrated in a Heto Power Dry LL 3000 lyophiliser under standard conditions for 72 h, obtaining about 70 g dry weight (30% active material), with a concentration of $1,2 \times 10^{11}$ CFU/g p.s. In such conditions a product with a cell viability close to 90%, stable, easy to store and handle is obtained, which at operational doses of $5 \times 10^7$ CFU/mL allows the preparation of about 140 L of a product ready to be applied on the crop plants to be protected. Subsequently, the material was packaged in vacuum sealed bags, and preserved at 4 °C, which maintains its shelf life for more than one year.

**Example 6. Evaluation of acute oral toxicity of the strain of the invention in rats**

Species: Wisatr Rat RjHan: WI, 8 weeks of age.

16 animals (8 female and 8 male)

Distribution: 2 males and 2 females in four groups: control day 3, control day 21, Pseudomonas azotoformans day 3 and Pseudomonas azotoformans day 21

Acclimatization period: 12 days before experimental phase

Animals were administered once by oral gavage previous a fasting period. After that, animals were observed periodically during the first 24 hours.

Body weight was registered once a week and at the end of the study. Animals were observed during 21 days after which they were euthanized with sodium pentobarbital and necropsy was performed (expect on groups euthanized on day 3)

Test item administration

Route: oral, by gastric gavage

Frequnc: single administration

Volume: 1ml

Administered dose: $2.53 \times 10^8$ CFU/ml = CFU/animal

No animals died during the study. No treatment-related clinical signs were found during the development of the study

All animals gained weight during experimental phase

No colony forming units were observed in any of the tissues organs and body fluids of control and treated rats on days 3 or 21 after administration

It was concluded that the test item *Pseudmonas azotoformans* does not cause adverse effects in rodents. The test item was cleared from bodied during first 3 days following administration without causing any adverse effect

REFERENCES CITED IN THE APPLICATION:

Arahal et al., Methods in Microbiology, Chapter 6, Whole genome analysis: Average Nucleotide Identity", Vol. 41 ISSN 0580-9517.

Xiang N et al. "Biological control of Meloidogyne incognita by Spore-forming Plant Growth-promoting Rhizobacteria on Cotton" Plant Disease 2017(101):774-784.

Schwyn B. and Neilands J. B. "Universal chemical assay for the detection and determination of siderophores." Anal Biochem, (1987) 160(1): 47-56.

Kuiper I. et al., "Characterization of two Pseudomonas putida lipopeptide biosurfactants, putisolvin I and II, which inhibit biofilm formation and break down existing biofilms". Mol Microbiol, 2004, 51: 97-113 López-García B. et al., "Identification of Novel Hexapeptides Bioactive against Phytopathogenic Fungi through Screening of a Synthetic Peptide Combinatorial Library". Appl Environ Microbiol, 2002, 68: 2453-2460

## Claims

1. A strain of *Pseudomonas sp.* deposited at the "Colección Española de Cultivos Tipo" (CECT) under the accession number CECT8708.

2. A process for obtaining a viable cell suspension derived from the strain as defined in Claim 1, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.

3. A process for obtaining a cell-free extract derived from the strain as defined in Claim 1, said process comprising: (i) inoculating the strain in a suitable culture medium, (ii) subjecting the inoculated culture medium to suitable growth conditions, (iii) separating the cells from the culture medium of step (ii), (iv) collecting cell-free extract; and (v) optionally subjecting the cell-free extract to a concentration process.

4. An inoculation product comprising the strain as defined in Claim 1.

5. A composition comprising the strain as defined in Claim 1 or the inoculation product as defined in claim 4, and one or more agriculturally acceptable compounds.

6. The composition according to Claim 5, wherein the one or more agriculturally acceptable compounds is selected from the group consisting of plant strengtheners, nutrients, wetting agents, compounds that improve adherence, buffering compounds, stabilisers, antioxidants, osmotic protectors and sunscreens.

7. The composition according to any of Claims 5 to 6, comprising at least one additional pesticide.

8. The composition according to Claim 7, wherein the additional pesticide is selected from the group consisting of a bacterial strain with antifungal and/or antibacterial activity, a fungicide, a bactericide, an herbicide, an insecticide or a nematicide.

9. Use of the strain as defined in Claim 1 or the inoculation product as defined in Claim 4, or the composition as defined in any of the Claims 5 to 8 as a pesticide in plants.

10. The use according to Claim 9 for controlling a disease caused by a bacterium or fungus in a plant.

11. A method for controlling an infection caused by bacterial or fungal pathogen in a plant, comprising applying the strain as defined in Claims 1, or the inoculation product as defined in Claim 4, or the composition as defined in any of the Claims 5 to 8, to the affected part of the plant and/or to the seed or to the substrate or soil used for growing said plant.

12. A kit comprising an effective amount of the strain as defined in claim 1, or the inoculation product as defined in Claim 4, or the composition as defined in any of the Claims 5 to 8.

13. Use of the kit according to claim 12 for controlling an infection caused by bacterial pathogens and/or fungal pathogens in a plant.

**Patentansprüche**

1. Ein Stamm von *Pseudomonas sp.* , hinterlegt bei der spanischen Kulturensammlung (CECT, *Colección Española de Cultivos Tipo*) unter der Hinterlegungsnummer CECT8708.

2. Ein Verfahren zum Erhalten einer lebensfähigen Zellsuspension, die von dem Stamm wie in Anspruch 1 definiert abgeleitet ist, wobei das Verfahren Folgendes umfasst: (i) Beimpfen des Stamms in einem Kulturmedium, (ii) Unterziehen des beimpften Kulturmediums aus Schritt (i) Bedingungen, die für das Wachstum des Stamms geeignet sind, und (iii) wahlweise Unterziehen des aus Schritt (ii) resultierenden Mediums einem Konzentrierungsschritt.

3. Ein Verfahren zum Erhalten eines zellfreien Extrakts, der von dem Stamm wie in Anspruch 1 definiert abgeleitet ist, wobei das Verfahren Folgendes umfasst: (i) Beimpfen des Stamms in einem geeigneten Kulturmedium, (ii) Unterziehen des beimpften Kulturmediums geeigneten Wachstumsbedingungen, (iii) Abtrennen der Zellen aus dem Kulturmedium aus Schritt (ii), (iv) Sammeln von zellfreiem Extrakt; und (v) wahlweise Unterziehen des zellfreien Extrakts einem Konzentrierungsverfahren.

4. Ein Inokulationsprodukt umfassend den Stamm wie in Anspruch 1 definiert.

5. Eine Zusammensetzung umfassend den Stamm wie in Anspruch 1 definiert oder das Impfprodukt wie in Anspruch 4 definiert und eine oder mehrere landwirtschaftlich akzeptable Verbindungen.

6. Die Zusammensetzung nach Anspruch 5, wobei die eine oder mehrere landwirtschaftlich akzeptablen Verbindungen aus der Gruppe bestehend aus Pflanzenstärkungsmitteln, Nährstoffen, Netzmitteln, Verbindungen, welche die Haftfähigkeit verbessern, Puffermitteln, Stabilisatoren, Antioxidationsmitteln, kompatiblen Soluten und Sonnenschutzmitteln ausgewählt sind.

7. Die Zusammensetzung nach einem der Ansprüche 5 bis 6, umfassend mindestens ein weiteres Pestizid.

8. Die Zusammensetzung nach Anspruch 7, wobei das zusätzliche Pestizid ausgewählt ist aus der Gruppe bestehend aus einem bakteriellen Stamm mit antimykotischer und/oder antibakterieller Wirkung, einem Antimykotikum, einem Bakterizid, einem Herbizid, einem Insektizid oder einem Nematizid.

9. Verwendung des Stamms wie in Anspruch 1 definiert oder des Impfprodukts wie in Anspruch 4 definiert, oder der Zusammensetzung wie in einem der Ansprüche 5 bis 8 definiert als Pestizid in Pflanzen.

10. Die Verwendung nach Anspruch 9 zur Bekämpfung einer durch ein Bakterium oder einen Pilz in einer Pflanze verursachten Krankheit.

11. Ein Verfahren zur Kontrolle einer Infektion, die durch bakterielle oder pilzliche Erreger in einer Pflanze verursacht wird, umfassend das Auftragen des Stamms wie in Anspruch 1 definiert, oder des Impfprodukts wie in Anspruch 4 definiert, oder der Zusammensetzung wie in einem der Ansprüche 5 bis 8 definiert, auf den betroffenen Teil der Pflanze und/oder auf das Saatgut oder auf das Substrat oder den Boden, das/der zum Züchten der Pflanze verwendet wird.

12. Ein Kit umfassend eine wirksame Menge des Stamms wie in Anspruch 1 definiert, oder des Impfprodukts wie in Anspruch 4 definiert, oder der Zusammensetzung wie in einem der Ansprüche 5 bis 8 definiert.

13. Verwendung des Kits nach Anspruch 12 zur Bekämpfung einer durch bakterielle Erreger und/oder pilzliche Erreger verursachten Infektion in einer Pflanze.

**Revendications**

1. Une souche de *Pseudomonas sp.* déposée à la Collection espagnole de cultures type (CECT, *Colección Española de Cultivos Tipo*) sous le numéro d'accession CECT8708.

2. Un procédé pour obtenir une suspension cellulaire viable dérivée de la souche telle que définie dans la revendication 1, le procédé comprenant : (i) inoculer la souche dans un milieu de culture, (ii) soumettre le milieu de culture inoculé de l'étape (i) à des conditions appropriées pour la croissance de la souche, et (iii) éventuellement soumettre le milieu résultant de l'étape (ii) à une étape de concentration.

3. Un procédé pour obtenir un extrait exempt de cellules dérivé de la souche telle que définie dans la revendication 1, ledit procédé comprenant : (i) inoculer la souche dans un milieu de culture approprié, (ii) soumettre le milieu de culture inoculé à des conditions de croissance appropriées, (iii) séparer les cellules du milieu de culture de l'étape (ii), (iv) collecter l'extrait exempt de cellules ; et (v) éventuellement soumettre l'extrait exempt de cellules à un processus de concentration.

4. Un produit d'inoculation comprenant la souche telle que définie dans la revendication 1.

5. Une composition comprenant la souche telle que définie dans la revendication 1 ou le produit d'inoculation tel que défini dans la revendication 4, et un ou plusieurs composés acceptables en agriculture.

6. La composition selon la revendication 5 dans laquelle le un ou les plusieurs composés acceptables en agriculture est choisi dans le groupe constitué des matériaux fortifiants de plantes, des nutriments, des agents mouillants, des composés qui améliorent l'adhérence, des composés tampons, des stabilisateurs, des antioxydants, des osmoprotecteurs et des protecteurs solaires.

7. La composition selon l'une quelconque des revendications 5 à 6 comprenant au moins un pesticide additionnel.

8. La composition selon la revendication 7, dans laquelle le pesticide additionnel est choisi dans le groupe constitué d'une souche bactérienne avec activité antifongique et/ou antibactérienne, un fongicide, un bactéricide, un herbicide, un insecticide ou un nématicide.

9. Utilisation de la souche telle que définie dans la revendication 1 ou du produit d'inoculation tel que défini dans la revendication 4, ou de la composition telle que définie dans l'une quelconque des revendications 5 à 8 en tant que pesticide chez les plantes.

10. L'utilisation selon la revendication 9 pour contrôler une maladie causée par une bactérie ou un champignon dans une plante.

11. Un procédé pour contrôler une infection causée par un agent pathogène bactérien ou fongique chez une plante, comprenant l'application de la souche telle que définie dans la revendication 1, ou du produit d'inoculation tel que défini dans la revendication 4, ou de la composition telle que définie dans l'une quelconque des revendications 5 à 8, sur la partie affectée de la plante et/ou sur la semence ou sur le substrat ou le sol utilisé pour la culture de ladite plante.

12. Un kit comprenant une quantité efficace de la souche telle que définie dans la revendication 1, ou du produit d'inoculation tel que défini dans la revendication 4, ou de la composition telle que définie dans l'une quelconque des revendications 5 à 8.

13. Utilisation du kit selon la revendication 12 pour contrôler une infection causée par des agents pathogènes bactériens et/ou des agents pathogènes fongiques chez une plante.

FIG.1

FIG. 2

B

FIG.3

H$_2$O　　　　KingBmod　　　B2021　　　DSM 18862

**REFERENCES CITED IN THE DESCRIPTION**

**Patent documents cited in the description**

- EP 19382190
- WO 2017178529 A1
- US 5232850 A
- WO 9403065 A1

**Non-patent literature cited in the description**

- **ARAHAL et al.** Whole genome analysis: Average Nucleotide Identity. *Methods in Microbiology*, vol. 41
- **BANKEVICH, A et al.** SPAdes: a new genome assembly algorithm and its applications to single-cell sequencing.. *Journal of Computational Biology*, 2012, vol. 19 (5), 455-477
- **SCHWYN B. ; NEILANDS J. B.** Universal chemical assay for the detection and determination of siderophores.. *Anal Biochem*, 1987, vol. 160 (1), 47-56
- **KUIPER I. et al.** Characterization of two Pseudomonas putida lipopeptide biosurfactants, putisolvin I and II, which inhibit biofilm formation and break down existing biofilms. *Mol Microbiol*, 2004, vol. 51, 97-113
- **LÓPEZ-GARCÍA B. et al.** Identification of Novel Hexapeptides Bioactive against Phytopathogenic Fungi through Screening of a Synthetic Peptide Combinatorial Library. *Appl Environ Microbiol*, 2002, vol. 68, 2453-2460
- **ARAHAL et al.** Methods in Microbiology. *Whole genome analysis: Average Nucleotide Identity*, vol. 41
- **XIANG N et al.** Biological control of Meloidogyne incognita by Spore-forming Plant Growth-promoting Rhizobacteria on Cotton. *Plant Disease*, 2017 (101), 774-784